# EUROPEAN PATENT APPLICATION

(11) **EP 4 691 470 A1**
(43) Date of publication of application: **11.02.2026**
(21) Application number: 24784348.5
(22) Date of filing: 03.04.2024
(51) Int. Cl.: A61K 31/519, A61P 43/00

(54) **USE OF HETEROCYCLIC COMPOUND IN TREATMENT OF INTERSTITIAL PNEUMONIA**

(30) Priority: 04.04.2023 CN 202310354802
(71) Applicant: SUZHOU KINTOR PHARMACEUTICALS, INC, Suzhou, Jiangsu 215123 (CN)
(72) Inventor: TONG, Youzhi, Suzhou, Jiangsu 215123 (CN)
(74) Representative: Cabinet Becker et Associés
(86) International application number: PCT/CN2024/085810
(87) International publication number: WO 2024/208266

(57) **Abstract**

A heterocyclic compound having a hedgehog pathway antagonist activity, especially a compound having a structure of formula A or a pharmaceutically acceptable salt, solvate, hydrate, stereoisomer, tautomer and prodrug thereof. The compound can effectively block the Shh signaling pathway, can down-regulate the expression of the pulmonary fibrosis-related protein **α-SMA** protein, and can significantly inhibit the progression of pulmonary fibrosis lesions.

## Description

### Citation of Related Applications

The present invention claims the priority of patent application No. 2023103548021 titled "use of heterocyclic compound in treatment of interstitial pneumonia" filed in China on April 4, 2023, the content of which is incorporated herein by reference in its entirety.

### Technical Field

The present application belongs to the field of drugs and relates to the use of a drug in treating interstitial pneumonia, and specifically relates to the use of a heterocyclic compound having hedgehog pathway antagonist activity in treating interstitial pneumonia.

### Background Art

Interstitial pneumonia is an inflammatory and fibrotic disease of the pulmonary interstitium caused by various factors. The lesions primarily affect the pulmonary interstitium and alveolar spaces, ultimately leading to fibrosis of the pulmonary interstitium and resulting in the loss of alveolar-capillary function. Based on etiology, it is mainly classified into interstitial pneumonia of known causes and idiopathic interstitial pneumonia. Idiopathic interstitial pneumonia is a type of interstitial lung disease of unknown causes, the most common clinical manifestation is idiopathic pulmonary fibrosis.

Idiopathic pulmonary fibrosis (IPF) is a progressive, fatal fibrotic lung disease of unknown etiology. It is a chronic inflammatory reactive interstitial lung disease characterized pathologically by diffuse alveolitis and pulmonary fibrosis. Clinically, it manifests as early acute lower respiratory tract infection, leading to pulmonary arterial hypertension, progressive dyspnea, and ultimately, death predominantly due to respiratory failure. In the injured lungs of the early stage of pulmonary fibrosis, substantial infiltration of inflammatory cells such as macrophages, neutrophils, and eosinophils can be observed. These inflammatory cells produce a large amount of cytokines such as TNF-α, TGF-β, and IFN-y. These cytokines can mediate fibroproliferation, activate inflammatory cells, and this persistent inflammation promotes the migration of local fibroblasts and accelerates their transformation into myofibroblasts.

IPF is a rare disease, affecting approximately 1.6 million people worldwide in 2020, accounting for 20-50% of all interstitial lung diseases. In May 2018, China's "First Batch of Rare Disease Catalogue" designated IPF as one of the 121 rare diseases.

In 2014, the U.S. FDA approved the first drugs in the United States for the treatment of IPF: Nintedanib from Boehringer Ingelheim Pharmaceuticals Inc. and Pirfenidone from InterMune Inc. Although Nintedanib and Pirfenidone have demonstrated significant beneficial effects in slowing down the progression of IPF, neither of them can prevent or even reverse or cure the symptoms of IPF for patients with mild or moderate forced vital capacity impairment. Furthermore, the most significant side effects associated with Nintedanib and Pirfenidone are gastrointestinal problems, particularly diarrhea, nausea, abdominal pain, decreased appetite, and weight loss. Additionally, when the two drugs are used in combination, the gastrointestinal side effects will accumulate.

The Hedgehog (Hh) pathway is a highly conserved intercellular signaling system composed of the Hh ligand, two transmembrane receptor proteins-Patched (PTCH) and Smoothened (SMO), and downstream transcription factor Gli proteins. Humans have three Hedgehog homolog genes: Sonic hedgehog (Shh), Indian hedgehog (Ihh), and Desert hedgehog (Dhh).

Shh signaling is crucial for embryonic lung development, but this pathway is basically in a dormant state in healthy adult lung tissue. The reactivation of the Shh signaling pathway is a characteristic of fibrotic lung tissue in IPF, affecting the increased migration and proliferation of fibroblasts. Many non-clinical studies have shown that Shh signaling pathway plays a crucial role in idiopathic pulmonary fibrosis. It has been reported that in IPF tissues, the expression of genes or proteins such as the Smoothened transmembrane receptor (SMO) and the downstream transcription factor Gli1 is higher than in normal lung tissue. Moreover, stimulation of lung fibrotic cells isolated from the lung tissue of IPF patients with Shh leads to increased expression of SMO and Gli1 proteins and genes. Preclinical *in vivo* experiments have shown that in mice with SMO gene knockout, both lung injury and the degree of pulmonary fibrosis were improved to a certain extent in bleomycin-induced pulmonary fibrosis model.

Patent WO2018/006756A1 discloses a chiral heterocyclic compound of formula II having hedgehog pathway antagonist activity, and a pharmaceutically acceptable salt, stereoisomer, or solvate thereof. The compound of formula II blocks the hedgehog pathway, thereby inhibiting abnormal cell growth and blocking the metastasis and regeneration of tumor cells, and exhibits favorable biological activity, pharmacokinetic properties, and excellent pharmacological characteristics.

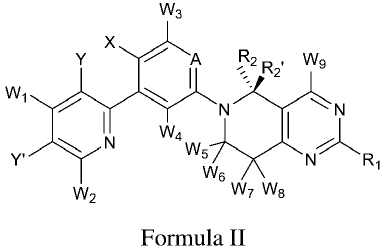

### Summary

During the experimental research, the applicant was surprised to discover that compound A, as an SMO inhibitor, can effectively treat or prevent pulmonary fibrosis. That is, this application provides a drug for treating or preventing interstitial pneumonia. This drug inhibits the SMO signaling pathway and can simultaneously down-regulate the expression of the pulmonary fibrosis-related protein α-SMA (α-smooth muscle actin) and significantly improves the severity of pulmonary fibrosis and idiopathic pulmonary fibrosis.

The objectives of the present application are achieved by the following technical solutions.

In a first aspect, the present application provides a compound represented by formula A or a pharmaceutically acceptable salt, solvate, hydrate, and prodrug thereof in the preparation of a drug for treating interstitial pneumonia.

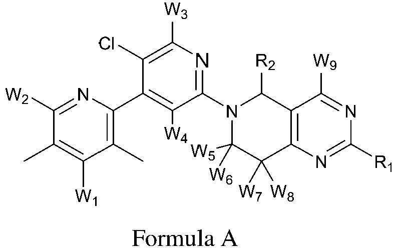

Wherein,
R₂ is C₁₋₃ alkyl, CD₃ or CF₃;
R₁ is -NRₓR_{y}, wherein Rₓ and R_{y} are independently H, alkyl, cycloalkyl, alkylcycloalkyl, C(O)R", or -NRₓR_{y} may together form a 4-7 membered heterocyclic ring, wherein the 4-7 membered heterocyclic ring is substituted or unsubstituted;
R" is C₁₋₅ alkyl;
W₁, W₂, W₃, W₄, W₅, W₆, W₇, W₈ and W₉ are independently H or D.

In some embodiments of the present application, R₁ in the compound of Formula A is -NRₓR_{y}, which together form a 4-7 membered heterocyclic ring, wherein the 4-7 membered heterocyclic ring is substituted or unsubstituted; further preferably, the compound of formula A includes the following compounds:

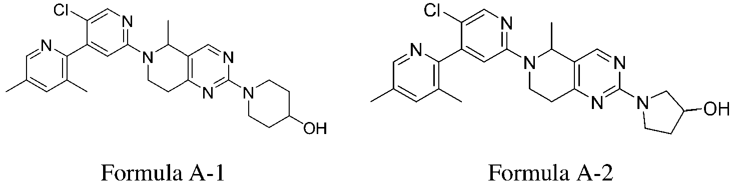

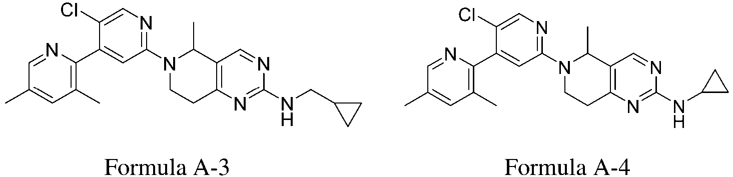

In some embodiments of the present application, the compound represented by formula A is a compound having a structure of formula B:

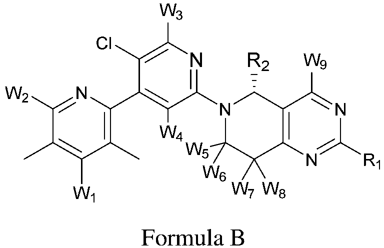

Wherein, R₂ is C₁₋₃ alkyl, CD₃ or CF₃;
R₁ is -NRₓR_{y}, wherein Rₓ and R_{y} are independently H, alkyl, cycloalkyl, alkylcycloalkyl, C(O)R", or -NRₓR_{y} may together form a 4-7 membered heterocyclic ring, wherein the 4-7 membered heterocyclic ring is substituted or unsubstituted;
R" is C₁₋₅ alkyl;
W₁, W₂, W₃, W₄, W₅, W₆, W₇, W₈ and W₉ are independently H or D.

Preferably, R₁ in the compound of Formula B is -NRₓR_{y}, which together form a 4-7 membered heterocyclic ring, wherein the 4-7 membered heterocyclic ring is substituted or unsubstituted; further preferably, the compound of formula B is a compound comprising the following compounds, preferably a compound having the structure of formula B-1.

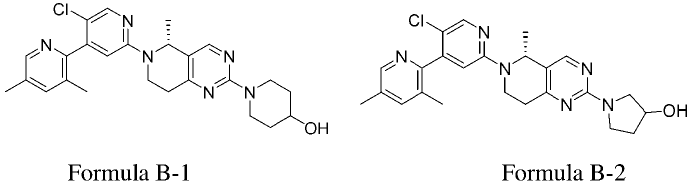

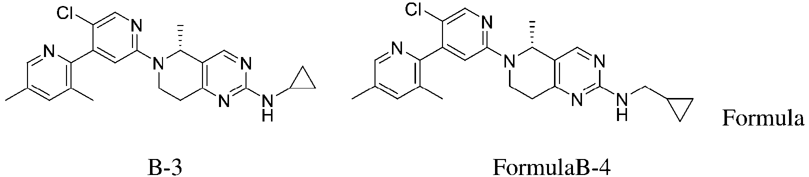

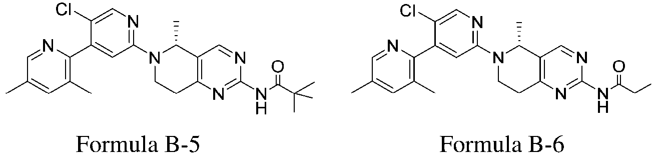

In some embodiments of the present application, the compound represented by formula A is a compound having a structure of formula C:

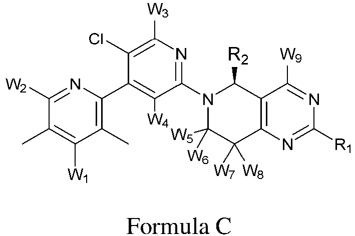

Wherein, R₂ is C₁₋₃ alkyl, CD₃ or CF₃;
R₁ is -NRₓR_{y}, wherein Rₓ and R_{y} are independently H, alkyl, cycloalkyl, alkylcycloalkyl, C(O)R", or -NRₓR_{y} may together form a 4-7 membered heterocyclic ring, wherein the 4-7 membered heterocyclic ring is substituted or unsubstituted;
R" is C₁₋₅ alkyl;
W₁, W₂, W₃, W₄, W₅, W₆, W₇, W₈ and W₉ are independently H or D.

Preferably, R₁ in the compound of Formula C is -NRₓR_{y}, which together form a 4-7 membered heterocyclic ring, wherein the 4-7 membered heterocyclic ring is substituted or unsubstituted; further preferably, the compound of formula C has a structure of formula C-1.

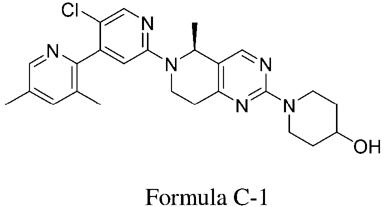

In some embodiments of the present application, the drug is a pharmaceutically acceptable salt of the compound of formula A, preferably a hydrochloride, a succinate or a fumarate of the compound of formula A.

Preferably, the drug is a pharmaceutically acceptable salt of a compound of formula A-1, formula B -1 or formula C -1; preferably a hydrochloride salt, a succinate salt or a fumarate salt of the compound of formula A-1, formula B-1 or formula C-1, more preferably a fumarate salt of the compound of formula A-1 or formula B-1; In the fumarate of formula A-1, the molar ratio of formula A-1 to fumaric acid is 1: 0.2-1, preferably 1: 0.3, 1: 0.5, 1: 0.6, 1: 0.8, 1: 1; In the fumarate of formula B-1, the molar ratio of formula B-1 to fumaric acid is 1: 0.2-1, preferably 1: 0.3, 1: 0.5, 1: 0.6, 1: 0.8, 1: 1; more preferably 1: 0.3, 1: 0.5, 1: 0.6.

In some embodiments of the present application, the drug is a crystal form of the fumarate of the compound of formula B-1.

In some embodiments of the present application, the drug is crystal form A of the fumarate of the compound of formula B-1.

The fumarate crystal form A of the compound of formula B-1 provided in the present application has X-ray powder diffraction pattern comprising characteristic peaks at 2θ values of 6.0±0.2°, 12.9±0.2°, and 16.2±0.2°; furthermore, the fumarate crystal form A of the compound of formula B-1 provided in the present application has X-ray powder diffraction pattern further comprising characteristic peaks at one or two or three of 20 values of 13.3 ± 0.2°, 15.4 ± 0.2°, 19.0 ± 0.2°; preferably, the fumarate crystal form A of the compound of formula B-1 provided in the present application has X-ray powder diffraction pattern further comprising characteristic peaks at one or two or three of 2θ values of 12.2 ± 0.2°, 18.1 ± 0.2°, 20.7 ± 0.2°; more preferably, the fumarate crystal form A of the compound of formula B-1 provided in the present application has X-ray powder diffraction pattern comprising characteristic peaks at 20 values of 6.0 ± 0.2°, 6.5 ± 0.2°, 9.9 ± 0.2°, 12.2 ± 0.2°, 12.9 ± 0.2°, 13.3 ± 0.2°, 13.7 ± 0.2°, 15.4 ± 0.2°, 16.2 ± 0.2°, 17.6 ± 0.2°, 18.1 ± 0.2°, 19.0 ± 0.2°, 19.6 ± 0.2°, and 20.7 ± 0.2°.

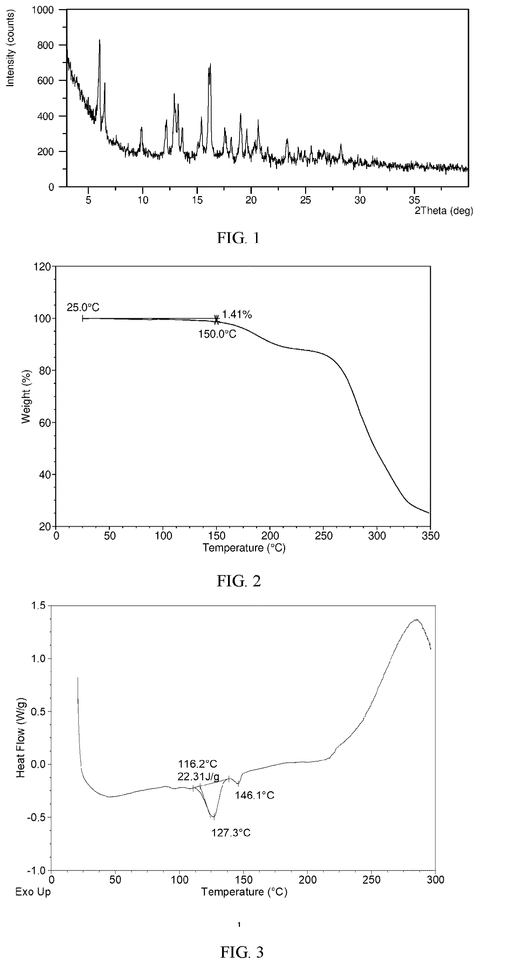

Furthermore, the fumarate crystal form A of the compound of formula B-1 provided in the present application has an X-ray powder diffraction pattern substantially as shown in FIG.1, and/or has diffraction peaks substantially as shown in Table 1-1.

The fumarate crystal form A of the compound of formula B-1 provided in the present application, when subjected to thermogravimetric analysis, has a weight loss gradient of about 1.4 ± 0.5% (eg, 1.4 ± 0.2%) when heated to 150°C. Preferably, the thermogravimetric analysis diagram is basically as shown in FIG. 2.

The fumarate crystal form A of the compound of formula B-1 provided in the present application has an endothermic peak at 127.3 ± 2°C (eg, 127.3 ± 1°C.) and 146.1 ± 2°C (eg, 146.1 ± 1°C.) when performing differential scanning calorimetry analysis. Preferably, the differential scanning calorimetry analysis diagram is basically as shown in FIG.3.

The fumarate crystal form A of the compound of formula B-1 provided in the present application, wherein the molar ratio of the compound of formula B-1 to the fumaric acid is 1: 0.5.

The fumarate crystal form A of the compound of formula B-1 provided in the present application is anhydrous compound.

The present application further provides a method for preparing the fumarate crystal form A of the compound of formula B-1, the method comprising: stirring the compound of formula B-1 and fumaric acid in a mixed solvent of an alkylketone and an alkane, filtering and drying to obtain the fumarate crystal form A.

Furthermore, the molar ratio of the compound of formula B-1 to the fumaric acid in the preparation method is 1: 0.3~1.5, preferably 1: 0.3, 1: 0.4, 1: 0.5, 1: 0.6, 1: 0.7, 1: 0.8, 1: 0.9, 1: 1.0, 1: 1.5, and more preferably 1: 0.5~0.7.

Furthermore, the volume ratio of the alkylketone to the alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 3~5.

Furthermore, the alkylketone may include acetone, butanone, methyl isobutyl ketone and cyclohexanone; the alkane may include isopentane, n-pentane, cyclopentane, n-hexane, cyclohexane, n-heptane, isooctane, and petroleum ether; the mixed solvent of the alkylketone and the alkane is preferably a mixed solvent of acetone and n-heptane.

Furthermore, the temperature during stirring is -5~40°C, preferably 5-35°C, more preferably room temperature.

Furthermore, the stirring is performed under a dark condition.

Furthermore, the stirring time is 1 h~8 d, preferably 6 h ~5 d, and more preferably 12 h ~1 d.

Furthermore, the mixture may be stirred at room temperature for 1~4 d, and then stirred at 0 ~10°C for 1~4 d.

In some embodiments of the present application, the drug is crystal form B of the fumarate of the compound of formula B-1.

The fumarate crystal form B of the compound of formula B-1 provided in the present application has X-ray powder diffraction pattern comprising characteristic peaks at 2θ values of 13.2±0.2°, 16.0±0.2°, 18.6±0.2°; furthermore, the fumarate crystal form B of the compound of formula B-1 provided in the present application has X-ray powder diffraction pattern further comprising characteristic peaks at one or two or three of 12.7 ± 0.2°, 15.4 ± 0.2°, 16.6 ± 0.2°; preferably, the fumarate crystal form B of the compound of formula B-1 provided in the present application has X-ray powder diffraction pattern further comprising characteristic peaks at one or two or three of 17.2 ± 0.2°, 18.0 ± 0.2°, 20.4 ± 0.2°; more preferably, the fumarate crystal form B of the compound of formula B-1 provided in the present application has X-ray powder diffraction pattern comprising characteristic peaks at 2θ values of 12.7 ± 0.2°, 13.2 ± 0.2°, 15.4 ± 0.2°, 16.0 ± 0.2°, 16.6 ± 0.2°, 17.2 ± 0.2°, 18.0 ± 0.2°, 18.6 ± 0.2°, 19.0 ± 0.2°, 20.4 ± 0.2°, 22.8 ± 0.2°, 26.8 ± 0.2°, 28.8 ± 0.2°, 29.4 ± 0.2°.

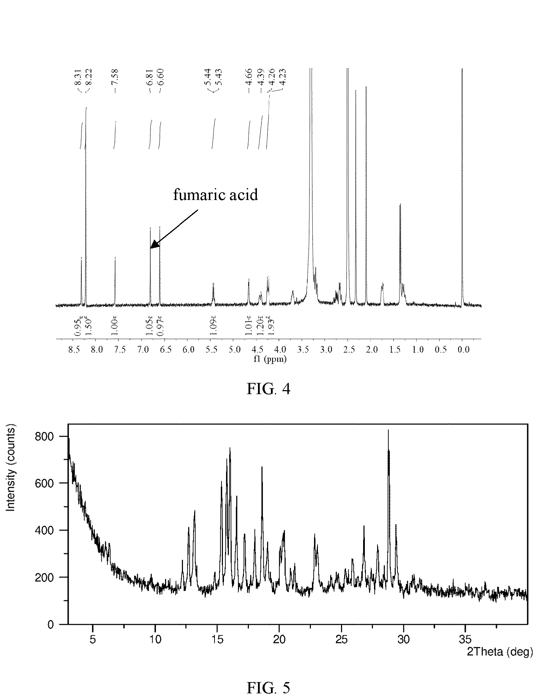

Furthermore, the fumarate crystal form B of the compound of formula B-1 provided in the present application has X-ray powder diffraction pattern substantially as shown in FIG. 5, and/or has a diffraction peak substantially as shown in Table 1-2.

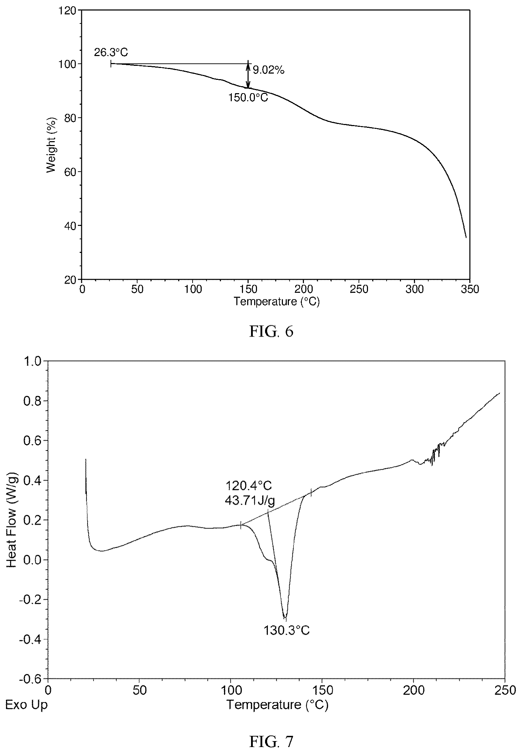

The fumarate crystal form B of the compound of formula B-1 provided in the present application, when subjected to thermogravimetric analysis, has a weight loss gradient of about 9.0 ± 0.5% (eg, 9.0 ± 0.2%) when heated to 150°C. Preferably, the thermogravimetric analysis diagram is basically as shown in FIG. 6.

The fumarate crystal form B of the compound of formula B-1 provided in the present application has an endothermic peak at 130.3 ± 2°C (eg, 130.3 ± 1°C.) when performing differential scanning calorimetry analysis. Preferably, the differential scanning calorimetry analysis diagram is basically as shown in FIG. 7.

The fumarate crystal form B of the compound of formula B-1 provided in the present application, wherein the molar ratio of the compound of formula B-1 to the fumaric acid is 1: 0.6.

The fumarate crystal form B of the compound of formula B-1 provided in the present application is anhydrous compound.

The present application further provides a method for preparing the fumarate crystal form B of the compound of formula B-1, the method comprising: stirring the compound of formula B-1 and fumaric acid in a mixed solvent of an ester and an alkane, filtering, and drying to obtain the fumarate crystal form B.

Furthermore, the molar ratio of the compound of formula B-1 to the fumaric acid in the preparation method is 1: 0.3 -1.5, preferably 1: 0.3, 1: 0.4, 1: 0.5, 1: 0.6, 1: 0.7, 1: 0.8, 1: 0.9, 1: 1.0, 1: 1.5, and more preferably 1: 0.5~0.7.

Furthermore, the volume ratio of the ester to the alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 3~5.

Furthermore, the ester may comprise ethyl acetate and isopropyl acetate; the alkane may comprise isopentane, n-pentane, cyclopentane, n-hexane, cyclohexane, n-heptane, isooctane, and petroleum ether; the mixed solvent of the ester and the alkane is preferably a mixed solvent of ethyl acetate and cyclohexane.

Furthermore, the temperature during stirring is 5~40°C, preferably 15~35°C, more preferably room temperature.

Furthermore, the stirring is performed under a dark condition.

Furthermore, the stirring time is 0.5~10d, preferably 1~8d, more preferably 1~4d.

In some embodiments of the present application, the drug is crystal form C of the fumarate of the compound of formula B-1.

The fumarate crystal form C of the compound of formula B-1 provided in the present application has X-ray powder diffraction pattern comprising characteristic peaks at 2θ values of 15.1 ± 0.2°, 17.1 ± 0.2°, 19.4 ± 0.2°; furthermore, the fumarate crystal form C of the compound of formula B-1 provided in the present application has X-ray powder diffraction pattern further comprising characteristic peaks at one or two or three of 11.0 ± 0.2°, 17.8 ± 0.2°, 21.7 ± 0.2°; preferably, the fumarate crystal form C of the compound of formula B-1 provided in the present application has X-ray powder diffraction pattern further comprising characteristic peaks at one or two or three of 5.0 ± 0.2°, 10.1 ± 0.2°, 27.1 ± 0.2°; more preferably, the fumarate crystal form C of the compound of formula B-1 provided in the present application has X-ray powder diffraction pattern comprising characteristic peaks at 20 values of 5.0 ± 0.2°, 10.1 ± 0.2°, 11.0 ± 0.2°, 12.9 ± 0.2°, 15.1 ± 0.2°, 16.1 ± 0.2°, 17.1 ± 0.2°, 17.8 ± 0.2°, 19.4 ± 0.2°, 20.2 ± 0.2°, 21.7 ± 0.2°, 27.1 ± 0.2°.

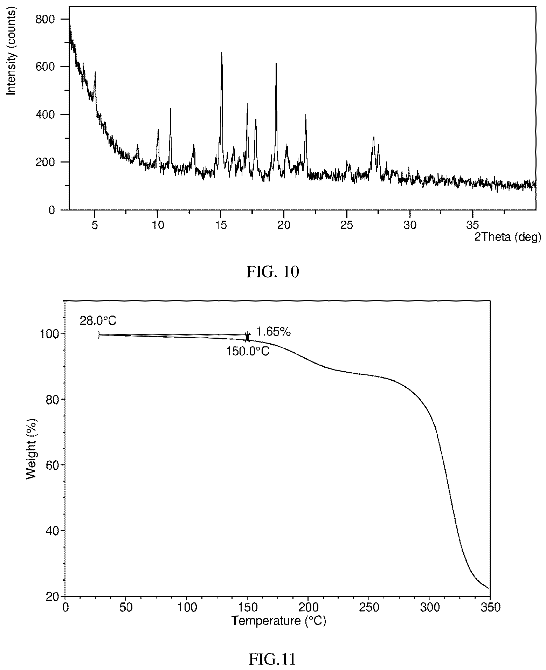

Furthermore, the fumarate crystal form C of the compound of formula B-1 provided in the present application has an X-ray powder diffraction pattern substantially as shown in FIG.10, and/or has diffraction peaks substantially as shown in Table 1-3.

The fumarate crystal form C of the compound of formula B-1 provided in the present application, when subjected to thermogravimetric analysis, has a weight loss gradient of about 1.7±0.5% (eg, 1.7±0.2%) when heated to 150°C. Preferably, the thermogravimetric analysis diagram is basically as shown in FIG. 11.

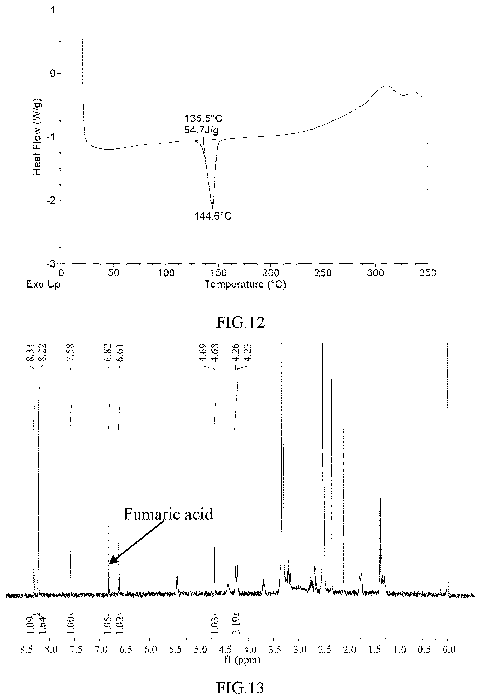

The fumarate crystal form C of the compound of formula B-1 provided in the present application has an endothermic peak at 144.6 ± 2°C (eg, 144.6 ± 1°C.) when performing differential scanning calorimetry analysis. Preferably, the differential scanning calorimetry analysis diagram is basically as shown in FIG. 12.

The fumarate crystal form C of the compound of formula B-1 provided in the present application, wherein the molar ratio of the compound of formula B-1 to the fumaric acid is 1: 0.5.

The fumarate crystal form C of the compound of formula B-1 provided in the present application is anhydrous compound.

The present application further provides a method for preparing the fumarate crystal form C of the compound of formula B-1, the method comprising: stirring a compound of formula B-1 and fumaric acid, or a fumarate salt of the compound of formula B-1 in a linear or branched C2 -C6 nitrile, filtering, and drying to obtain the fumarate crystal form C.

Furthermore, the fumarate salt of the compound of formula B-1 comprises the fumarate crystal form A of the compound of formula B-1.

Furthermore, the linear or branched C2 -C6 nitrile may comprise acetonitrile.

Furthermore, the temperature during stirring is -5~40°C, preferably 5~30°C, preferably room temperature.

Furthermore, the stirring is performed under a dark condition.

Furthermore, the stirring time is 6h~12d, preferably 10h~8d, and more preferably 12h ~ 6d.

Furthermore, when the compound of formula B-1and the fumaric acid are used as raw materials, stirring may be performed at room temperature for 6~18 h, and then stirred at 0~10°C for 10~20 h.

In some embodiments of the present application, the drug is crystal form D of the fumarate of the compound of formula B-1.

The fumarate crystal form D of the compound of formula B-1 provided in the present application has X-ray powder diffraction pattern comprising characteristic peaks at 2θ values of 12.7 ± 0.2°, 17.6 ± 0.2°, 19.1 ± 0.2°; furthermore, the fumarate crystal form D of the compound of formula B-1 provided in the present application has X-ray powder diffraction pattern further comprising characteristic peaks at one or two or three of 6.3 ± 0.2°, 13.2 ± 0.2°, 16.1 ± 0.2°; preferably, the fumarate crystal form D of the compound of formula B-1 provided in the present application has X-ray powder diffraction pattern further comprising characteristic peaks at one or two or three of 17.2 ± 0.2°, 14.0 ± 0.2°, 20.9 ± 0.2°; more preferably, the fumarate crystal form D of the compound of formula B-1 provided in the present application has characteristic peaks at 20 values of 6.3 ± 0.2°, 11.3 ± 0.2°, 12.7 ± 0.2°, 13.2 ± 0.2°, 14.0 ± 0.2°, 16.1 ± 0.2°, 17.2 ± 0.2°, 17.6 ± 0.2°, 19.1 ± 0.2°, 20.5 ± 0.2°, 20.9 ± 0.2° and 24.1 ± 0.2°.

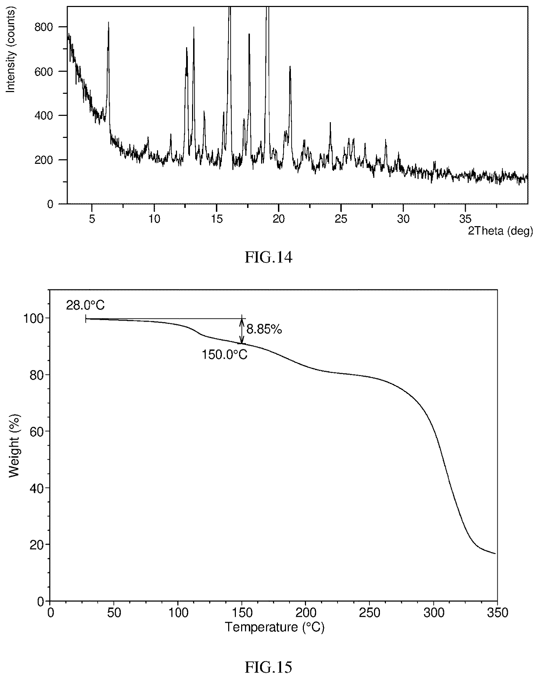

Furthermore, the fumarate crystal form D of the compound of formula B-1 provided in this application has X-ray powder diffraction pattern substantially as shown in FIG. 14, and/or has a diffraction peak substantially as shown in Tables 1-4.

The fumarate crystal form D of the compound of formula B-1 provided in the present application, when subjected to thermogravimetric analysis, has a weight loss gradient of about 8.9 ± 0.5% (eg, 8.9 ± 0.2%) when heated to 150°C. Preferably, the thermogravimetric analysis diagram is basically as shown in FIG. 15.

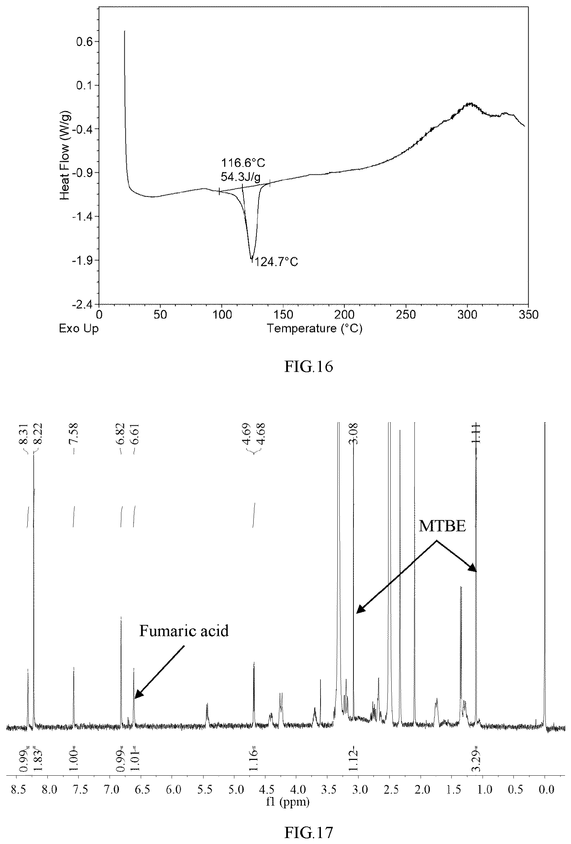

The fumarate crystal form D of the compound of formula B-1 provided in the present application has an endothermic peak at 124.7 ± 2°C (eg, 124.7 ± 1°C.) when performing differential scanning calorimetry analysis. Preferably, the differential scanning calorimetry analysis diagram is basically as shown in FIG. 16.

The fumarate crystal form D of the compound of formula B-1 provided in the present application, wherein the molar ratio of the compound of formula B-1 to the fumaric acid is 1: 0.5.

The present application further provides a method for preparing the fumarate crystal form D of the compound of formula B-1, the method comprising: stirring a fumarate salt of the compound of formula B-1 in ether, or a mixed solvent of a cyclic amide and an aromatic hydrocarbon, filtering, and drying to obtain the fumarate crystal form D.

Furthermore, the fumarate salt of the compound of formula B-1 comprises the fumarate crystal form A of the compound of formula B-1.

Furthermore, the ether may comprise methyl tert-butyl ether, tetrahydrofuran or 2-methyltetrahydrofuran, preferably methyl tert-butyl ether; the cyclic amide may include N-methylpyrrolidone, and the aromatic hydrocarbon may include benzene, toluene, xylene; the mixed solvent of the cyclic amide and the aromatic hydrocarbon is preferably a mixed solvent of N-methylpyrrolidone and toluene.

Furthermore, the temperature during stirring is-5~40°C, preferably 0-30°C, more preferably 0~10°C, or room temperature.

Furthermore, the volume ratio of the cyclic amide to the aromatic hydrocarbon is 1: 3~15, preferably 1: 3, 1: 4, 1: 5, 1: 6, 1: 7, 1: 8, 1: 9, 1: 10, 1: 11, 1: 12, 1: 13, 1: 14, 1: 15, more preferably 1: 7~11.

Furthermore, the stirring is performed under a dark condition.

Furthermore, the stirring time is 1~15 d, preferably 3~10d, more preferably 5~8d.

In some embodiments of the present application, the drug is crystal form E of the fumarate of the compound of formula B-1.

The fumarate crystal form E of the compound of formula B-1 provided in the present application has X-ray powder diffraction pattern comprising characteristic peaks at 2θ values of 15.2 ± 0.2°, 16.0 ± 0.2°, 22.0 ± 0.2°; furthermore, the fumarate crystal form E of the compound of formula B-1 provided in the present application has X-ray powder diffraction pattern further comprising characteristic peaks at one or two or three of 12.8 ± 0.2°, 20.5 ± 0.2°, 26.0 ± 0.2°; preferably, the fumarate crystal form E of the compound of formula B-1 provided in the present application has X-ray powder diffraction pattern further comprising characteristic peaks at one or two or three of 16.8 ± 0.2°, 18.1 ± 0.2°, 19.7 ± 0.2°; more preferably, the fumarate crystal form E of the compound of formula B-1 provided in the present application has X-ray powder diffraction pattern comprising characteristic peaks at 20 values of 12.8 ± 0.2°, 15.2 ± 0.2°, 16.0 ± 0.2°, 16.8 ± 0.2°, 18.1 ± 0.2°, 19.7 ± 0.2°, 20.5 ± 0.2°, 22.0 ± 0.2°, 23.9 ± 0.2°, 26.0 ±0.2°.

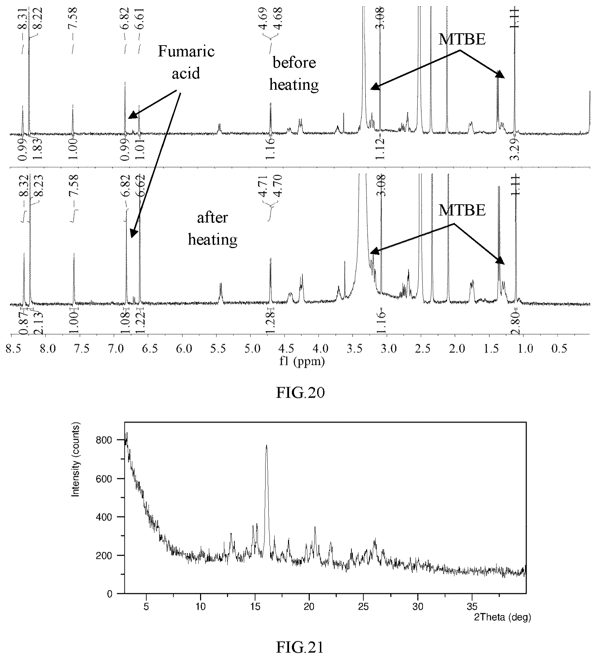

Furthermore, the fumarate crystal form E of the compound of formula B-1 provided in the present application has X-ray powder diffraction pattern substantially as shown in FIG. 21, and/or has a diffraction peak substantially as shown in Table 1-5.

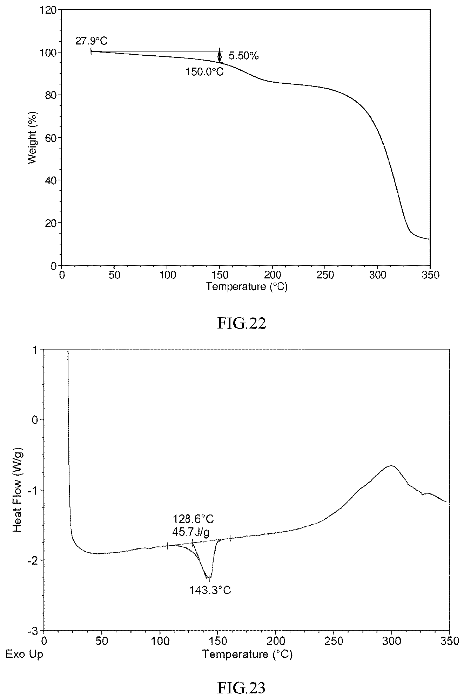

The fumarate crystal form E of the compound of formula B-1 provided in the present application, when subjected to thermogravimetric analysis, has a weight loss gradient of about 5.5 ± 0.5% (eg, 5.5 ± 0.2%) when heated to 150°C. Preferably, the thermogravimetric analysis diagram is basically as shown in FIG. 22.

The fumarate crystal form E of the compound of formula B-1 provided in the present application has an endothermic peak at 143.3 ± 2°C (eg, 143.3 ± 1°C.) when performing differential scanning calorimetry analysis. Preferably, the differential scanning calorimetry analysis diagram is basically as shown in FIG. 23.

The fumarate crystal form E of the compound of formula B-1 provided in the present application, wherein the molar ratio of the compound of formula B-1 to the fumaric acid is 1: 0.5.

The fumarate crystal form E of the compound of formula B-1 provided in the present application is hydrate or anhydrous crystal form that is hygroscopic (surface water adsorption).

The present application further provides a method for preparing the fumarate crystal form E of the compound of formula B-1, the method comprising: stirring a fumarate salt of the compound of formula B-1 in a mixed solvent of an alkyl alcohol and an alkane, filtering, and drying to obtain the fumarate crystal form E.

Furthermore, the fumarate salt of the compound of formula B-1 comprises the fumarate crystal form A of the compound of formula B-1.

Furthermore, the alkyl alcohol may comprise methanol, ethanol, n-propanol, and isopropanol; the alkane may comprise isopentane, n-pentane, cyclopentane, n-hexane, cyclohexane, n-heptane, isooctane and petroleum ether; the mixed solvent of the alkyl alcohol and the alkane is preferably a mixed solvent of isopropanol and n-heptane.

Furthermore, the volume ratio of the alkyl alcohol to the alkane is 1: 1~6, preferably 1: 1, 1: 2, 1: 3, 1: 4, 1: 5, 1: 6, more preferably 1: 3~5.

Furthermore, the temperature during stirring is 15~40°C, preferably room temperature.

Furthermore, the stirring is performed under a dark condition.

Furthermore, the stirring time is 1~10 d, preferably 2~8 d, more preferably 3~6 d.

In some embodiments of the present application, the drug is crystal form F of the fumarate of the compound of formula B-1.

The fumarate crystal form F of the compound of formula B-1 provided in the present application has X-ray powder diffraction pattern comprising characteristic peaks at 2θ values of 11.1 ± 0.2°, 16.6 ± 0.2°, 22.4 ± 0.2°; furthermore, the fumarate crystal form F of the compound of formula B-1 provided in the present application has X-ray powder diffraction pattern further comprising characteristic peaks at one or two or three of 16.9 ± 0.2°, 18.6 ± 0.2°, 21.0 ± 0.2°; preferably, the fumarate crystal form F of the compound of formula B-1 provided in the present application has X-ray powder diffraction pattern further comprising characteristic peaks at one or two or three of 5.5 ± 0.2°, 14.5 ± 0.2°, 25.0 ± 0.2°; more preferably, the fumarate crystal form F of the compound of formula B-1 provided in the present application has characteristic peaks at 20 values of 5.5 ± 0.2°, 11.1 ± 0.2°, 14.5 ± 0.2°, 15.5 ± 0.2°, 16.6 ± 0.2°, 16.9 ± 0.2°, 17.5 ± 0.2°, 18.6 ± 0.2°, 21.0 ± 0.2°, 22.4 ± 0.2°, 23.3 ± 0.2°, 25.0 ± 0.2°.

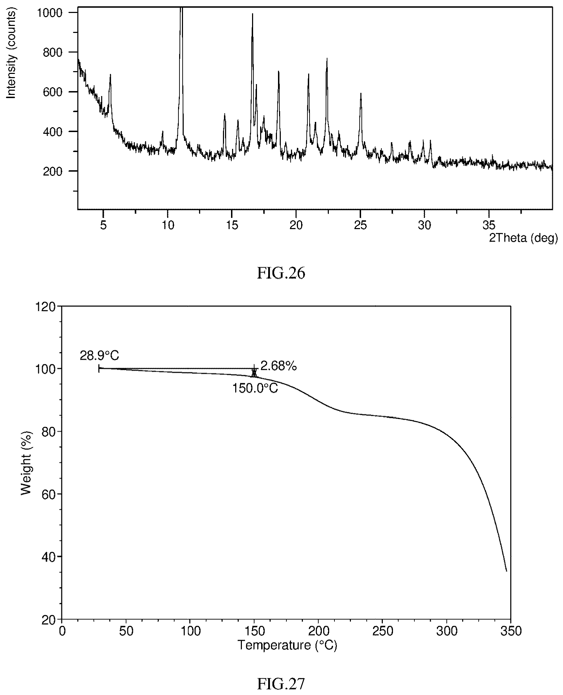

Furthermore, the fumarate crystal form F of the compound of formula B-1 provided in the present application has X-ray powder diffraction pattern substantially as shown in FIG. 26, and/or has a diffraction peak substantially as shown in Table 1-6.

The fumarate crystal form F of the compound of formula B-1 provided in the present application, when subjected to thermogravimetric analysis, has a weight loss gradient of about 2.7 ± 0.5% (eg, 2.7 ± 0.2%) when heated to 150°C. Preferably, the thermogravimetric analysis diagram is basically as shown in FIG. 27.

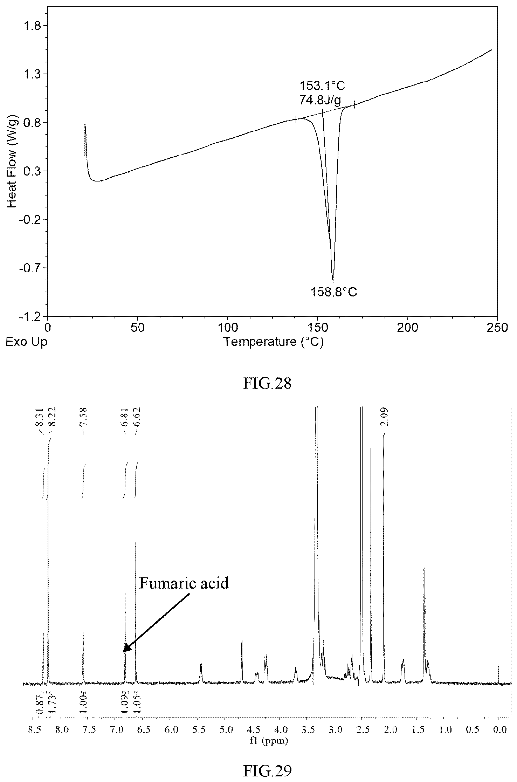

The fumarate crystal form F of the compound of formula B-1 provided in the present application has an endothermic peak at 158.8 ± 2°C (eg, 158.8 ± 1°C.) when performing differential scanning calorimetry analysis. Preferably, the differential scanning calorimetry analysis diagram is basically as shown in FIG. 28.

The fumarate crystal form F of the compound of formula B-1 provided in the present application, wherein the molar ratio of the compound of formula B-1 to the fumaric acid is 1: 0.5.

The fumarate crystal form F of the compound of formula B-1 provided in the present application is anhydrous compound.

The present application also provides a method for preparing the fumarate crystal form F of the compound of formula B-1, wherein the method is selected from any one of the following:
i. stirring the compound of formula B-1 and fumaric acid in an organic solvent, filtering and drying to obtain;
ii. stirring the compound of formula B-1 in an organic solvent, filtering and drying to obtain.

Furthermore, in method i, the molar ratio of the compound of formula B-1 to the fumaric acid is 1: 0.3~1.5, preferably 1: 0.3, 1: 0.4, 1: 0.5, 1: 0.6, 1: 0.7, 1: 0.8, 1: 0.9, 1: 1.0, 1: 1.5, and more preferably 1: 0.5~0.7.

Furthermore, in method i, the organic solvent comprises a mixed solvent of alkyl ketone and alkane, C2 -C6 nitrile, ether, a mixed solvent of alkyl alcohol and alkane, and a mixed solvent of alkyl alcohol and water.

Furthermore, the alkyl ketone may comprise acetone, butanone, methyl isobutyl ketone, and cyclohexanone; the linear or branched C2-C6 nitrile may comprise acetonitrile; the ether may comprise methyl tert-butyl ether, tetrahydrofuran, 2-methyltetrahydrofuran, 1,4-dioxane, preferably methyl tert-butyl ether; the alkyl alcohol may comprise methanol, ethanol, n-propanol, isopropanol; the alkane may comprise isopentane, n-pentane, cyclopentane, n-hexane, cyclohexane, n-heptane, isooctane, and petroleum ether; the mixed solvent of the alkyl ketone and the alkane is preferably a mixed solvent of acetone and n-heptane; the mixed solvent of the alkyl alcohol and the alkane is preferably a mixed solvent of isopropanol and n-heptane; and the mixed solvent of the alkyl alcohol and the water is preferably a mixed solvent of ethanol and water.

Furthermore, in the mixed solvent of alkyl ketone and alkane, the volume ratio of alkyl ketone to alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 2~5; in the mixed solvent of alkyl alcohol and alkane, the volume ratio of alkyl alcohol to alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 3~5; in a mixed solvent of alkyl alcohol and water, the volume ratio of alkyl alcohol to water is 1: 3~15, preferably 1: 3, 1: 4, 1: 5, 1: 6, 1: 7, 1: 8, 1: 9, 1: 10, 1: 11, 1: 12, 1: 13, 1: 14, 1: 15, more preferably 1: 7~11.

Furthermore, in method i, in the case of using the mixed solvent, the order of adding compound of formula B-1, fumaric acid, and solvent is not limited. For example, after the organic solvent forming the mixed solvent can be prepared into a mixed solvent in proportion, the compound of formula B-1 and fumaric acid are added, or the compound of formula B-1 and fumaric acid can also be added to one of the solvents forming the mixed solvent, and then the rest of the solvent is added.

Furthermore, in method i, the temperature during stirring is 0~70°C, preferably 15°C, 20°C, 25°C, 30°C 35°C 40°C, 45°C, 50°C, 55°C, 60°C, 65°C, 70°C, more preferably room temperature or 45~55°C.

Furthermore, in method i, stirring is performed under a dark condition.

Furthermore, in method i, the stirring time is 6 h~9 d, preferably 6 h, 12 h, 1 d, 2 d, 3 d, 4 d, 5 d, 6 d, 7 d, 8 d, 9 d, and more preferably 2~6 d.

Furthermore, in method ii, the fumarate salt of the compound of formula B-1 comprises a fumarate crystal form A, a crystal form B, or a crystal form C.

Furthermore, in method ii, the organic solvent is selected from alkyl ketone, C2-C6 nitrile, and ether; furthermore, the alkyl ketone may comprise acetone, butanone, methyl isobutyl ketone, cyclohexanone, preferably acetone, methyl isobutyl ketone; the C2-C6 nitrile may comprise acetonitrile; the ether may comprise methyl tert-butyl ether, tetrahydrofuran, 2-methyltetrahydrofuran, 1,4-dioxane, preferably methyl tert-butyl ether;
furthermore, in method ii, the temperature during stirring is 15~70°C., preferably 15°C, 20°C., 25°C, 30°C, 35°C, 40°C, 45°C., 50°C, 55°C, 60°C, 65°C, 70°C, more preferably room temperature, or 45~55°C.

Furthermore, in method ii, stirring is performed under a dark condition.

Furthermore, in method ii, the stirring time is 0.5~12 d, preferably 0.5 d, 1d, 2d, 3d, 4d, 5d, 6d, 7d, 8d, 9d, 10d, 11d, 12d, more preferably 0.5~8 d.

In some embodiments of the present application, the drug is fumarate crystal form G of the compound of formula B-1.

The fumarate crystal form G of the compound of formula B-1 provided in the present application has X-ray powder diffraction pattern comprising characteristic peaks at 2θ values of 15.6 ± 0.2°, 17.7± 0.2°, 20.6± 0.2°.

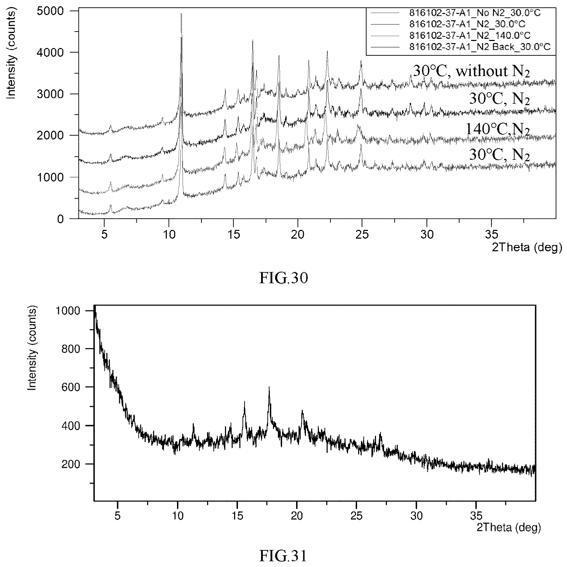

Furthermore, the fumarate crystal form G of the compound of formula B-1 provided in the present application has X-ray powder diffraction pattern substantially as shown in FIG. 31, and/or has a diffraction peak substantially as shown in Table 1-10.

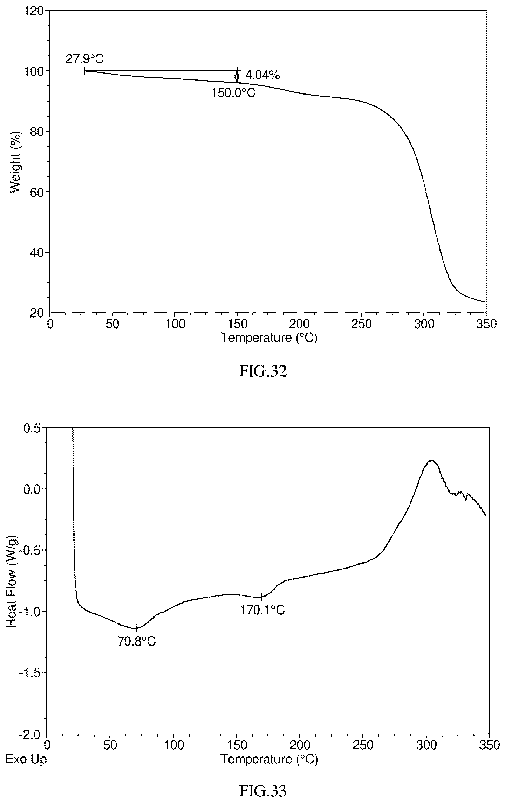

The fumarate crystal form G of the compound of formula B-1 provided in the present application, when subjected to thermogravimetric analysis, has a weight loss gradient of about 4.0 ± 0.5% (eg, 4.0 ± 0.2%) when heated to 150°C. Preferably, the thermogravimetric analysis diagram is basically as shown in FIG. 32.

The fumarate crystal form G of the compound of formula B-1 provided in the present application has an endothermic peak at 70.8 ± 2°C (eg, 70.8 ± 1°C) and 170.1 ± 2°C, eg, 170.1 ± 1°C) when performing differential scanning calorimetry analysis. Preferably, the differential scanning calorimetry analysis diagram is basically as shown in FIG. 33.

The fumarate crystal form G of the compound of formula B-1 provided in the present application, wherein the molar ratio of the compound of formula B-1 to the fumaric acid is 1: 0.3.

The present application further provides a method for preparing the fumarate crystal form G of the compound of formula B-1, the method comprising: stirring a fumarate salt of the compound of formula B-1 in a mixed solvent of alkyl alcohol and water, or a mixed solvent of a cyclic amide and water, filtering, and drying to obtain.

Furthermore, the fumarate salt of the compound of formula B-1 comprises a fumarate crystal form A of the compound of formula B-1.

Furthermore, the alkyl alcohol may comprise methanol, ethanol, n-propanol, isopropanol, preferably ethanol; the cyclic amide may comprise N-methylpyrrolidone; the mixed solvent of the alkyl alcohol and the water is preferably a mixed solvent of ethanol and water; and the mixed solvent of the cyclic amide and the water is preferably a mixed solvent of N-methylpyrrolidone and water.

Furthermore, the volume ratio of the alkyl alcohol to water is 1: 3~15, preferably 1: 3, 1: 4, 1: 5, 1: 6, 1: 7, 1: 8, 1: 9, 1: 10, 1: 11, 1: 12, 1: 13, 1: 14, 1: 15, more preferably 1: 7~11.

Furthermore, the volume ratio of the cyclic amide to water is 1: 3~15, preferably 1: 3, 1: 4, 1: 5, 1: 6, 1: 7, 1: 8, 1: 9, 1: 10, 1: 11, 1: 12, 1: 13, 1: 14, 1: 15, more preferably 1: 7~11.

Furthermore, the temperature during stirring is 15~70°C, preferably 45~55°C.

Furthermore, the stirring is performed under a dark condition.

Furthermore, the stirring time is 1~10 d, preferably 2~8 d, more preferably 3~7 d.

In some embodiments of the present application, the drug is crystal form H of the fumarate of the compound of formula B-1.

The fumarate crystal form H of the compound of formula B-1 provided in the present application has X-ray powder diffraction pattern comprising characteristic peaks at 2θ values of 12.4 ± 0.2°, 16.2 ± 0.2°, 21.8 ± 0.2°; furthermore, the fumarate crystal form H of the compound of formula B-1 provided in the present application has X-ray powder diffraction pattern further comprising characteristic peaks at one or two or three of 12.8 ± 0.2°, 15.8 ± 0.2°, 18.0 ± 0.2°; preferably, the fumarate crystal form H of the compound of formula B-1 provided in the present application has X-ray powder diffraction pattern further comprising characteristic peaks at one or two or three of 14.5 ± 0.2°, 16.9 ± 0.2°, 21.2 ± 0.2°; more preferably, the fumarate crystal form H of the compound of formula B-1 provided in the present application has characteristic peaks at 20 values of 5.2 ± 0.2°, 12.4 ± 0.2°, 12.8 ± 0.2°, 14.5 ± 0.2°, 15.8 ± 0.2°, 16.2 ± 0.2°, 16.9 ± 0.2°, 18.0 ± 0.2°, 20.3 ± 0.2°, 21.2 ± 0.2°, 21.8 ± 0.2°, 23.7 ± 0.2°.

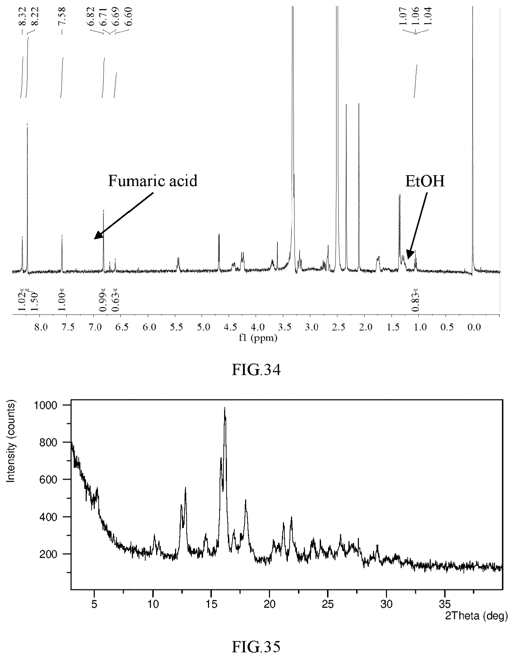

Furthermore, the fumarate crystal form H of the compound of formula B-1 provided in the present application, the X-ray powder diffraction pattern thereof is substantially as shown in FIG. 35, and/or has a diffraction peak substantially as shown in Table 1-11.

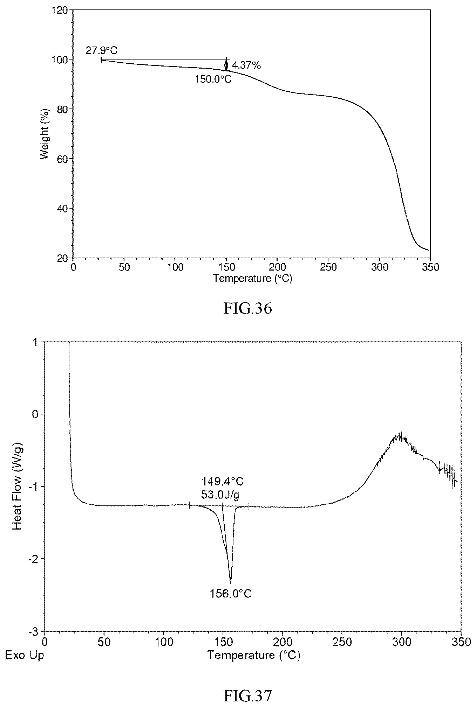

The fumarate crystal form H of the compound of formula B-1 provided in the present application, when subjected to thermogravimetric analysis, has a weight loss gradient of about 4.4 ± 0.5% (eg, 4.4 ± 0.2%) when heated to 150°C. Preferably, the thermogravimetric analysis diagram is basically as shown in FIG. 36.

The fumarate crystal form H of the compound of formula B-1 provided in the present application has an endothermic peak at 156.0 ± 2°C (eg, 156.0 ± 1°C.) when performing differential scanning calorimetry analysis. Preferably, the differential scanning calorimetry analysis diagram is basically as shown in FIG. 37.

The fumarate crystal form H of the compound of formula B-1 provided in the present application, wherein the molar ratio of the compound of formula B-1 to the fumaric acid is 1: 0.5.

The fumarate crystal form H of the compound of formula B-1 provided in the present application is hydrate or anhydrous crystal form that is hygroscopic (surface water adsorption).

The present application further provides a method for preparing the fumarate crystal form H of the compound of formula B-1, the method comprising: stirring a fumarate salt of the compound of formula B-1 in a mixed solvent of an alkyl alcohol and an alkane, filtering, and drying to obtain.

Furthermore, the fumarate salt of the compound of formula B-1 comprises the fumarate crystal form A of the compound of formula B-1.

Furthermore, the alkyl alcohol may comprise methanol, ethanol, n-propanol, and isopropanol; the alkane may comprise isopentane, n-pentane, cyclopentane, n-hexane, cyclohexane, n-heptane, isooctane, and petroleum ether; and the mixed solvent of the alkyl alcohol and the alkane is preferably a mixed solvent of isopropanol and n-heptane.

Furthermore, the volume ratio of the alkyl alcohol to the alkane is 1: 7-15, preferably 1: 7, 1: 8, 1: 9, 1: 10, 1: 11, 1: 12, 1: 13, 1: 14, 1: 15, more preferably 1: 7~11.

Furthermore, the temperature during stirring is 15~70°C, preferably 45~55°C.

Furthermore, the stirring is performed under a dark condition.

Furthermore, the stirring time is 1~10 d, preferably 2~8 d, more preferably 3~7 d.

In some embodiments of the present application, the drug is in the form of a crystal form of the succinate of the compound of formula B-1.

In some embodiments of the present application, the drug is succinate crystal form A of the compound of formula B-1.

The succinate crystal form A of the compound of formula B-1 provided in the present application has X-ray powder diffraction pattern comprising characteristic peaks at 2θ values of 22.1 ± 0.2° and 27.2 ± 0.2°.

Furthermore, the succinate crystal form A of the compound of formula B-1 provided in the present application has X-ray powder diffraction pattern comprising diffraction peaks substantially as shown in Table 2-1.

The present application further provides a method for preparing the succinate crystal form A of the compound of formula B-1, the method comprising: stirring the compound of formula B-1 and succinic acid in an ether, filtering, and drying to obtain.

Furthermore, the ether may comprise methyl tert-butyl ether, tetrahydrofuran, 2-methyltetrahydrofuran, 1,4-dioxane, preferably methyl tert-butyl ether.

Furthermore, the molar ratio of the compound of formula B-1 to succinic acid is 1: 0.3~3.0, preferably 1: 0.5~2.0, more preferably 1: 0.8~1.2.

Furthermore, the reaction comprises stirring the compound of formula B-1 and succinic acid in methyl tert-butyl ether, stirring at 0~10°C for 1~4 days, stirring for 1~3 days at -25~15°C, and then volatilizing at room temperature.

Furthermore, the stirring is performed under a dark condition.

In some embodiments of the present application, the drug is succinate crystal form B of the compound of formula B-1.

The succinate crystal form B of the compound of formula B-1 provided in the present application has X-ray powder diffraction pattern comprising characteristic peaks at 2θ values of 9.5 ± 0.2°, 14.4 ± 0.2°, 23.6 ± 0.2°; furthermore, the succinate crystal form B of the compound of formula B-1 provided in the present application has X-ray powder diffraction pattern further comprising characteristic peaks at one or two or three of 15.1 ± 0.2°, 19.2± 0.2°, 25.5 ± 0.2°; preferably, the succinate crystal form B of the compound of formula B-1 provided in the present application has X-ray powder diffraction pattern further comprising characteristic peaks at one or two or three of 15.6 ± 0.2°, 22.3 ± 0.2°, 24.0 ± 0.2°; more preferably, the succinate crystal form B of the compound of formula B-1 provided in the present application has X-ray powder diffraction pattern comprising characteristic peaks at 2θ values of 9.5 ± 0.2°, 12.2 ± 0.2°, 14.4 ± 0.2°, 15.1 ± 0.2°, 15.6 ± 0.2°, 16.5 ± 0.2°, 19.2 ± 0.2°, 22.3 ± 0.2°, 23.6 ± 0.2°, 24.0 ± 0.2°, 25.5 ± 0.2°, 27.3 ± 0.2°.

Furthermore, the succinate crystal form B of the compound of formula B-1 provided in the present application has X-ray powder diffraction pattern comprising diffraction peaks substantially as shown in Table 2-2.

The present application further provides a method for preparing the succinate crystal form B of the compound of formula B-1, the method comprising: stirring the compound of formula B-1 and succinic acid in a mixed solvent of alkyl ketone and alkane, filtering and drying to obtain.

Furthermore, the alkyl ketone may comprise acetone, butanone, methyl isobutyl ketone, and cyclohexanone; the alkane comprises isopentane, n-pentane, cyclopentane, n-hexane, cyclohexane, n-heptane, isooctane, and petroleum ether; and the mixed solvent of the alkyl ketone and the alkane is preferably a mixed solvent of acetone and n-heptane.

Furthermore, the molar ratio of the compound of formula B-1 to succinic acid is 1: 0.3~3.0, preferably 1: 0.5~2.0, more preferably 1: 0.8~1.2.

Furthermore, the volume ratio of the alkyl ketone to the alkane is 1: 1~15, preferably 1: 1, 1: 2, 1: 3, 1: 4, 1: 5, 1: 6, 1: 7, 1: 8, 1: 9, 1: 10, 1: 11, 1: 12, 1: 13, 1: 14, 1: 15, more preferably 1: 3~5.

Furthermore, the temperature during stirring is 15~40°C, preferably room temperature.

Furthermore, the stirring is performed under a dark condition.

Furthermore, the stirring time is 1~8 d, preferably 2~7 d, and more preferably 4~6d.

In some embodiments of the present application, the drug is hydrochloride of the compound of formula B-1.

The present application further provides a method for preparing the hydrochloride of the compound of formula B-1, the method comprising: the compound of formula B-1 and HCl were stirred in an organic solvent, filtered and dried to obtain.

Furthermore, the organic solvent is an ester, which may comprise ethyl acetate, isopropyl acetate, preferably ethyl acetate.

In this application, the mixed solvent only means that it is a mixed solvent during the reaction process but does not mean that it must be used in the form of a mixed solvent. In this application, there are no restrictions on the use of mixed solvents. For example, the organic solvent forming the mixed solvent can be mixed in proportion to form a mixed solvent for use; or one or more solvents forming the mixed solvent can be added to the reactor first, and then the remaining solvent is added.

In some embodiments of the present application, the interstitial pneumonia includes hypersensitivity pneumoniae (HP), drug induced lung disease, connective tissue disease-related interstitial pneumonia, autoimmune lung damage caused by pneumoconiosis, idiopathic interstitial pneumonia; wherein the idiopathic interstitial pneumonia includes idiopathic pulmonary fibrosis, desquamative interstitial pneumonia (DIP), nonspecific interstitial pneumonia (NSIP), cryptogenic organometallic pneumonia (COP), respiratory bronchiolitis-associated interstitial lung disease (RBILD), lymphocyte interstitial pneumonia (LIP), and the like.

Preferably, the interstitial pneumonia is idiopathic pulmonary fibrosis, and more preferably, the idiopathic pulmonary fibrosis is accompanied by pulmonary hypertension, gastroesophageal reflux disease, obstructive sleep apnea or coronary artery disease.

In a second aspect, the present application provides a drug (a compound represented by formula A, B, C, A -1, B -1, C -1, or a pharmaceutically acceptable salt, solvate, hydrate, prodrug thereof, such as a hydrochloride, succinate or fumarate of formula B-1, crystal form A-H of the fumarate of the compound of formula B-1, and a succinate crystal form A or B of the compound of formula B-1) for treating interstitial pneumonia; preferably, the interstitial pneumonia is idiopathic interstitial pneumonia, and more preferably idiopathic pulmonary fibrosis.

In a third aspect, the present application provides a pharmaceutical composition for treating interstitial pneumonia, preferably, the interstitial pneumonia is idiopathic interstitial pneumonia, and more preferably idiopathic pulmonary fibrosis.

In some embodiments of the present application, the pharmaceutical composition comprises (i) any one of the compound represented by formula A, B, C, A-1, B-1, C-1, or the pharmaceutically acceptable salt, solvate, hydrate, prodrug thereof, or any one of hydrochloride, succinate or fumarate of formula B-1, or any one of crystal forms A-H of fumarate of the compound of formula B-1, or any one of succinate crystal form A or B of the compound of formula B-1, and (ii) a pharmaceutically acceptable carrier and/or excipient.

In some embodiments of the present application, the pharmaceutical composition comprises a compound represented by formula A or a pharmaceutically acceptable salt, solvate, hydrate, prodrug thereof and second active pharmaceutical component ; wherein the second active pharmaceutical component is N-acetylcysteine, pirfenidone, nintedanib, glucocorticoids (such as methylprednisolone, prednisone, fluticasone), immunosuppressants (such as cyclosporine, cyclophosphamide, azathioprine), endothelin receptor antagonists (such as macitentan, Bosentan), Ambrisentan), JNK inhibitors (Tanzisertib (CC-930)), Itraconazole, Vismodegib, hydroxycarbamide, Danazol, Amikacin, dornase alfa (Pulmonzyme), Tobramycin, Tiotropium Bromide, Treprostinil, Zileuton, Sararigib (IPI-926), or a composition thereof.

In a fourth aspect, the present application provides a method for treating interstitial pneumonia, preferably, the interstitial pneumonia is idiopathic interstitial pneumonia, and more preferably idiopathic pulmonary fibrosis.

In some embodiments of the present application, the method comprises administering to a subject in need thereof a therapeutically effective amount of the drug ((i) any one of the compound represented by formula A, B, C, A-1, B-1, C-1, or a pharmaceutically acceptable salt, solvate, hydrate, prodrug thereof, or any one of hydrochloride, succinate or fumarate of the compound of formula B-1,, or any one of crystal forms A-H of fumarate of the compound of formula B-1, or any one of succinate crystal form A or B of the compound of formula B-1) of the first aspect or the pharmaceutical composition of the third aspect.

The drug administration route in the present application is not limited to oral administration, parenteral administration, transdermal administration, wherein parenteral administration is not limited to intravenous injection, subcutaneous injection, intramuscular injection.

### The beneficial effects of the present application

The present application provides a chiral heterocyclic compound having a hedgehog pathway antagonist activity, that is, a compound having a structure of formula A or a pharmaceutically acceptable salt, solvate, hydrate, stereoisomer, tautomer and prodrug thereof. The compound can effectively block the SHH signal path, and can down-regulate the expression of α-SMA related to pulmonary fibrosis-related protein (alpha-SMA protein), significantly improving the degree of pulmonary fibrosis; compared with positive drug nintedanib (dosage of 100 mg/kg, QD), the compound of the present application, in particular, compound formula B-1-1 (dosage of 40 mg/kg, BID) has the same excellent effect in inhibiting the progression of pulmonary fibrosis lesions.

### Brief description of the drawings

FIG. 1 shows an XRPD pattern of fumarate crystal form A of formula B-1.
FIG. 2 shows a TGA diagram of fumarate crystal form A of formula B-1.
FIG. 3 shows a DSC diagram of fumarate crystal form A of formula B-1.
FIG. 4 shows a ¹H NMR graph of fumarate crystal form A of formula B-1.
FIG. 5 shows an XRPD pattern of fumarate crystal form B of formula B-1.
FIG. 6 shows a TGA diagram of fumarate crystal form B of formula B-1.
FIG. 7 shows a DSC diagram of fumarate crystal form B of formula B-1.
FIG. 8 shows a ¹H NMR graph of fumarate crystal form B of formula B-1.
FIG. 9 shows a variable temperature XRPD pattern of fumarate crystal form B of formula B-1
FIG. 10 shows an XRPD pattern of fumarate crystal form C of formula B-1.
FIG. 11 shows a TGA diagram of fumarate crystal form C of formula B-1.
FIG. 12 shows a DSC diagram of fumarate crystal form C of formula B-1.
FIG. 13 shows a ¹H NMR graph of fumarate crystal form C of formula B-1.
FIG. 14 shows an XRPD pattern of fumarate crystal form D of formula B-1.
FIG. 15 shows a TGA diagram of fumarate crystal form D of formula B-1.
FIG. 16 shows a DSC diagram of t fumarate crystal form D of formula B-1.
FIG. 17 shows a ¹H NMR graph of fumarate crystal form D of formula B-1.
FIG. 18 shows an XRPD stack of the fumarate crystal form D of formula B-1 before and after drying and heating.
FIG. 19 shows an TGA stack of fumarate crystal form D of formula B-1 before and after drying and heating.
FIG. 20 shows a ¹H NMR stack of fumarate crystal form D of formula B-1 before and after heating.
FIG. 21 shows an XRPD pattern of fumarate crystal form E of formula B-1.
FIG. 22 shows a TGA diagram of fumarate crystal form E of formula B-1.
FIG. 23 shows a DSC diagram of fumarate crystal form E of formula B-1.
FIG.24 shows a ¹H NMR graph of fumarate crystal form E of formula B-1.
FIG. 25 shows an XRPD stack of fumarate crystal form E of formula B-1 before and after drying.
FIG. 26 shows an XRPD pattern of fumarate crystal form F of formula B-1.
FIG. 27 shows a TGA diagram of fumarate crystal form F of formula B-1.
FIG. 28 shows a DSC diagram of fumarate crystal form F of formula B-1.
FIG. 29 shows a ¹H NMR graph of fumarate crystal form F of formula B-1.
FIG. 30 shows a variable temperature XRPD pattern of fumarate crystal form F of formula B-1
FIG. 31 shows an XRPD pattern of fumarate crystal form G of formula B-1.
FIG. 32 shows a TGA diagram of fumarate crystal form G of formula B-1.
FIG. 33 shows a DSC diagram of fumarate crystal form G of formula B-1.
FIG. 34 shows a ¹H NMR graph of fumarate crystal form G of formula B-1.
FIG. 35 shows an XRPD pattern of fumarate crystal form H of formula B-1.
FIG. 36 shows a TGA diagram of fumarate crystal form H of formula B-1.
FIG. 37 shows a DSC diagram of fumarate crystal form H of formula B-1.
FIG.38 shows a ¹H NMR graph of fumarate crystal form H of formula B-1.
FIG. 39 shows an XRPD stack of fumarate crystal form H of formula B-1 before and after drying and heating.
FIG. 40 shows a TGA stack of fumarate crystal form H of formula B-1 before and after drying and heating.
FIG. 41 shows an XRPD pattern of succinate crystal form A of the compound of formula B-1.
FIG. 42 shows an XRPD pattern of succinate crystal form B of the compound of formula B-1.
FIG. 43 shows degradation diagram of TGF-β-induced GLi1, GLi2, and α-SMA proteins by compound B-1-1.
FIG. 44 shows the changes in body weight of the animals in the sham group, the model group, the nintedanib-100 mg/kg group, the B-1-1-20 mg/kg group, and the B-1-1-40 mg/kg group during the experiment.
FIG. 45 shows the changes in the weight growth rates of the animals in the sham group, the model group, the nintedanib-100 mg/kg group, the B-1-1-20 mg/kg group, and the B-1-1-40 mg/kg group during the experiment.
FIG. 46 shows the staining (HE staining) of fibrotic lesions in the left lung;
   A: Sham group; B: Model group; C: Nintedanib-100 mg/kg group; D: Compound B-1-1-20 mg/kg group; E: Compound B-1-1-40 mg/kg group.
FIG. 47 shows histological changes of terminal bronchioles and small pulmonary arteries in pulmonary fibrotic lesions (HE staining, 200X);
   A: Sham group; B: Model group; C: Nintedanib-100 mg/kg group; D: Compound B-1-1-20 mg/kg group; E: Compound B-1-1-40 mg/kg group; a: Small pulmonary artery; b: Small pulmonary bronchiole.
FIG48. shows the histological changes of terminal bronchioles and small pulmonary arteries at the periphery of pulmonary fibrotic lesions (HE staining, 200X);
   A: Sham group; B: Model group; C: Nintedanib-100 mg/kg group; D: Compound B-1-1-20 mg/kg group; E: Compound B-1-1-40 mg/kg group; a: Small pulmonary artery; b: Small pulmonary bronchiole.
FIG. 49 shows alveolar tissue damage within pulmonary fibrotic lesions (HEstaining, 400X);
   A: Sham group; B: Model group; C: Nintedanib-100 mg/kg group; D: Compound B-1-1-20 mg/kg group; E: Compound B-1-1-40 mg/kg group.
FIG. 50 shows injury score of terminal bronchioles and small pulmonary arteries within pulmonary fibrotic lesion areas.
Figure 51 shows injury score of terminal bronchioles and small pulmonary arteries at the periphery of pulmonary fibrotic lesions.
FIG. 52 Staining of fibrotic lesions in the left lung (Masson staining);
   Wherein: A: Sham group; B: Model group; C: Nintedanib-100 mg/kg group; D: Compound B-1-1-20 mg/kg group; E: Compound B-1-1-40 mg/kg group.
FIG. 53 Structural changes of alveolar tissue within fibrotic lesions (Masson staining, 400X);
   A: Sham group; B: Model group; C: Nintedanib-100 mg/kg group; D: Compound B-1-1-20 mg/kg group; E: Compound B-1-1-40 mg/kg group.
FIG. 54 Pulmonary fibrosis score.

### Detailed Description of the Invention

### Definition

Unless otherwise defined, the terms used herein have the same meaning as commonly understood by one of ordinary skill in the art. The terms used in the specification are only used to describe particular embodiments and are not intended to limit the present application.

The term "solvate" refers to a substance formed by the binding of compound A (or the pharmaceutically acceptable salt thereof) to at least one solvent molecule through non-covalent intermolecular forces. The solvent may be water (i.e., a hydrate), and compound A may be associated with one or more water molecules (e.g., monohydrate, dihydrate, trihydrate, etc.). The solvent may also be an alcohol (e.g., methanol, ethanol, propanol, isopropanol, etc.), a diol (e.g., propylene glycol), an ether (e.g., diethyl ether), an ester (e.g., ethyl acetate), or any other suitable solvent; the solvate of formula A may also exist as a mixed solvate (i.e., associated with two or more different solvents).

The term "alkyl" refers to a straight or branched chain saturated aliphatic hydrocarbon. Alkyl groups include groups having from 1 to 8 carbon atoms (C1-8 alkyl), from 1 to 6 carbon atoms (C1-6 alkyl) and from 1 to 4 carbon atoms (C1-4 alkyl), including, for example, methyl, ethyl, propyl, isopropyl, n-butyl, sec-butyl, tert-butyl, pentyl, 2-pentyl, isopentyl, neopentyl, hexyl, 2-hexyl, 3-hexyl and 3-methylpentyl. In some instances, a substituent of an alkyl group is specifically indicated. For example, "cyanoalkyl" refers to an alkyl group substituted with at least one cyano substituent.

The term "cycloalkyl" is a group that comprises one or more saturated rings in which all ring members are carbon, including, for example, cyclopropyl, cyclobutyl, cyclopentyl, cyclohexyl, cycloheptyl, cyclooctyl, adamantyl. Cycloalkyl groups do not comprise an aromatic ring or a heterocyclic ring. Certain cycloalkyl groups are C3-7 cycloalkyl, in which the cycloalkyl group contains a single ring having from 3 to 7 ring members, all of which are carbon.

The term "alkylcycloalkyl" refers to a cycloalkyl group connected to another atom via an alkyl group, wherein the alkyl and cycloalkyl groups are as defined herein.

The term "heterocyclic" refers to a ring structure containing 3-12 ring atoms, in which at least one ring atom is carbon and at least one ring atom is a heteroatom selected from N, O, and S. A heterocyclic group may be aromatic or non-aromatic. Piperidine and oxetane are non-limiting examples of non-aromatic heterocycles. Thiazole and pyridine are non-limiting examples of aromatic heterocycles.

The term "substituted," as used herein, denote that a molecular moiety is covalently bonded to an atom within a molecule of interest. For example, a ring substituent may be a moiety such as a halogen, alkyl group, haloalkyl group or other group that is covalently bonded to an atom (preferably a carbon or nitrogen atom) that is a ring member. Substituents of aromatic groups are generally covalently bonded to a ring carbon atom. Similarly, a chain substituent may be a moiety such as a halogen, alkyl group, haloalkyl group or other group that is covalently bonded to an atom (preferably a carbon or nitrogen atom) that is a member of the chain.

The term "drug" refers to a product that includes an active ingredient and at least one pharmaceutically acceptable excipient. The drugs covered of the present application encompasses any drug prepared by mixing an active ingredients, additional active ingredients and pharmaceutically acceptable excipients.

The term "effective amount" denotes an amount of an active pharmaceutical ingredient that is sufficient to affect a disease or condition after the active pharmaceutical ingredient has been administered to an individual/subject/patient for the purpose of preventing, alleviating and/or treating the disease or condition. The "effective amount" may vary depending on the active pharmacological ingredient, the symptoms or severity of the disease or condition, the individual's/subject's/patient's personal situations (for example, age, gender, weight, etc.), and other factors.

The term "pharmaceutically acceptable carrier and/or excipient" refers to carriers and/or excipients that are pharmacologically and/or physiologically compatible with the subject and the active ingredient, which are well known in the art (see, for example, Remington's Pharmaceutical Sciences. Edited by Gennaro AR, 19th ed. Pennsylvania: Mack Publishing Company, 1995), and includes, but is not limited to, pH adjusters, surfactants, adjuvants, and ion strength enhancers.

Unless otherwise specified, the term "compound" refers to any specific compound disclosed herein and includes tautomers, positional isomers, geometric isomers, and, where applicable, stereoisomers, including optical isomers (enantiomers) and other stereoisomers (non-enantiomers), as well as pharmaceutically acceptable salts and derivatives (including prodrug forms) thereof. When used in context, the term "compound" typically refers not only to a single compound but may also include other compounds, such as stereoisomers, positional isomers, and/or optical isomers (including racemic mixtures), as well as specific enantiomers or enantiomer-enriched mixtures of the disclosed compounds. The term also refers to prodrug forms of the compound, which have been modified to facilitate administration and delivery of the compound to the active site.

The term "pharmaceutically acceptable salt" is used to describe the salt form of one or more compounds described herein, which is provided for the purpose of increasing the solubility of the compound in gastric juices in the gastrointestinal tract of a patient to promote dissolution and bioavailability of the compound. Pharmaceutically acceptable salts, when applicable, include salts derived from pharmaceutically acceptable inorganic or organic bases and acids. Suitable salts include those derived from alkali metals (e.g., potassium and sodium), alkaline earth metals (e.g., calcium, magnesium, and ammonium salts), and many other acids and bases well known in the pharmaceutical field.

The term "prodrug" refers to a functional derivative of the compound that is readily converted in vivo to the desired compound. Therefore, in the treatment methods described in this application, the term "administering" includes treating the various diseases described using the compounds specifically disclosed or using compounds that may not be specifically disclosed but are converted in vivo to specific compounds after administration to a patient. The conventional procedures for selecting and preparing suitable prodrug derivatives are described, for example, in "Design of Prodrugs," H. Bundgaard, Elsevier, 1985.

The compounds of the present application can form solvates with conventional organic solvents or hydrates with water, and such solvates or hydrates are also included within the scope of the present application.

The compounds of the present application include all tautomers and mixtures thereof in any proportion.

Unless otherwise specified, the term "substantially consistent with a figure" with respect to an XRPD pattern in this application means that at least 50%, or at least 60%, or at least 70%, or at least 80%, or at least 90%, or at least 95%, or at least 99% of the peaks in the XRPD pattern of a certain polymorph are present in the given XRPD pattern. Additionally, variations in representative peak positions (20) and relative peak intensities are taken into account. Peak positions may vary between different instruments and samples, typically by up to 0.1° to 0.2°. Furthermore, relative peak intensities may also vary due to differences between instruments, as well as crystallinity, preferred orientation, sample preparation, and other factors known to those skilled in the art.

Unless otherwise specified, the term "substantially consistent with a particular figure" with respect to DSC or TGA spectra as used in this application is also intended to encompass variations related to these analytical techniques that are known to those skilled in the art. For peaks with clear boundaries in DSC spectra, variations of up to ±0.2°C are typically observed, and for broad peaks, even larger variations (up to ±1°C, or up to ±5°C) may occur. For mass loss in a TGA spectrum, the mass loss detected will vary slightly from instrument to instrument and from sample to sample, depending on many factors, including sample preparation and instrumentation. Variations of up to ±1% or ±2% are typical.

"About", as the term is used herein, unless otherwise stated or implied by context, in connection with a numeric value or range of values to describe a particular property of a compound or composition, indicate that the value or range of values may deviate to an extent deemed reasonable to one of ordinary skill in the art while still describing the particular property. Reasonable deviations include those that are within the accuracy or precision of the instrument(s) used in measuring, determining or deriving the particular property. Specifically, the term "about" when used in this context, indicate that the numeric value or range of values can vary by 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1%, or 0.01% of the recited value or range of values, typically by 10% to 0.5 %, more typically by 5% to 1%, while still describing the particular property.

As used herein, unless the context indicates or specifies, the symbol "±" is used to indicate that the preceding value is subject to the error or deviation of the following value. Specifically, the expression "X±Y" (where X and Y are both numerical values) means that the error or deviation of the value X is Y, that is, (X-Y) to (X+Y) are within the range of X±Y.

As used herein, unless the context indicates or specifies, the tilde "~" is used to indicate a numerical range that is inclusive of the numerical values stated before and after it. Specifically, the expression "X to Y" (where X and Y are both numerical values) means "greater than X and less than Y".

As used herein, unless the context indicates or specifies, the term "substantially as shown" when referring to, for example, an XRPD pattern/chart, TGA pattern, DSC pattern, etc., refers to patterns/values that are not necessarily the same as those described herein, but that fall within the limits of experimental error or deviation when considered by one of ordinary skill in the art.

As used herein, unless the context indicates or specifies, the term "h" is a unit of time, meaning hour.

As used herein, unless the context indicates or specifies, the term "d" is a unit of time, meaning day.

As used herein, unless the context indicates or specifies, "room temperature" specifically refers to 23 ± 3°C.

The present application is further described below with reference to examples, but these examples are not intended to limit the scope of the present application.

The instruments and methods of detection used herein are as follows:

### X-ray Powder Diffraction (XRPD)

The XRPD pattern was collected on an X-ray powder diffraction analyzer produced by PANalytalcal or Bruker, and the scan parameters are shown in the table below.

### XRPD parameters

| Parameters | Instrument 1 | Instrument 2 | Instrument 3 |
|---|---|---|---|
| Model | Empyrean | X' Pert3 | X' Pert3 |
| X ray | Cu, Kα, Kα1 (Å): 1.540598, Kα2 (Å): 1.544426 | Cu, Kα, Kα1 (Å): 1.540598, Kα2 (Å): 1.544426 | Cu, Kα, Kα1 (Å): 1.540598, Kα2 (Å): 1.544426 |
| | Kα2/Kα1 intensity | Kα2/Kα1 intensity | Kα2/Kα1 intensity |
| | ratio: 0.50 | ratio: 0.50 | ratio: 0.50 |
| Setting of X-ray light tube | 45 kV, 40 mA | 45 kV, 40 mA | 45 kV, 40 mA |
| Divergent slit | Automatic | 1/8° | 1/8° |
| Mode of scanning | Continuous | Continuous | Continuous |
| Scan range (°2θ) | 3°-40° | 3°-40° | 3°-40° |
| Scan time per step (s) | 17.8 | 46.7 | 46.7 |
| Scan step (°2θ) | 0.0167 | 0.0263 | 0.0263 |
| Test time | ~5 min 30 s | ~5 min | ~5 min |

In the table, "~" denotes " about ".

### Thermogravimetric Analysis (TGA) and Differential Scanning Calorimetry (DSC)

TGA and DSC profiles were collected respectively on a TA Q500/5000/5500 thermal gravimetric analyzer and a TA Q200/2500 differential scanning calorimeter, and the test parameters were shown in table below.

### TGA and DSC parameters

| Parameters | TGA | DSC |
|---|---|---|
| Method | Linear heating up | Linear heating up |
| Sample plate | Aluminum pan, open | Aluminum pan, cover/uncover |
| Temperature range | Room Temperature-Set the end point temperature | 25 °C -Set the end point temperature |
| Scan rate (°C/ min) | 10 | 10 |
| Protective gas | Nitrogen | Nitrogen |

### mDSC parameters

| Parameters | Set value |
|---|---|
| Test Mode | Conventional mDSC |
| Amplitude | 1.0 °C |
| Modulation period | 60 s |
| Scan rate | 3.0 °C/min |
| Protective gas | N₂ at 50.00 mL/min |

### Dynamic Vapor Sorption (DVS)

The DVS curves were collected on a DVS Intrinsic from SMS (Surface Measurement Systems).The relative humidity at 25°C was corrected with the deliquescence points of LiCl, Mg(NO₃)₂ and KCl. The DVS test parameters were shown in table below.

### DVS Parameters

| **Parameters** | **Set value** |
|---|---|
| temperature | 25 °C |
| Sample amount | 10-20 mg |
| Protective gas and flow rate | Nitrogen, 200 mL/min |
| dm/dt | 0.002 %/min |
| Minimum dm/dt balance time | 10 min |
| Maximum dm/dt balance time | 180 min |
| RH range | 0 %RH-95 %RH |
| | 10 % (0 %RH-90 %RH, 90 %RH-0 %RH) |
| RH gradient | 5 % (90% RH-95 %RH, 95 %RH-90 %RH) |

### Solution NMR

The liquid nuclear magnetic hydrogen spectrum was collected on a Bruker 400M nuclear magnetic resonance instrument, and DMSO-d6 was used as a solvent.

### High Performance Liquid Chromatography (HPLC)

The purity test, the dynamic solubility and the stability test are tested by the Agilent 1260/1100 high performance liquid chromatograph, and the analysis conditions and chiral purity test analysis conditions of the high performance liquid chromatography test are respectively as follows.

### HPLC Parameters

| Instrument | Agilent 1260,DVD/VWD detector | |
|---|---|---|
| Chromatographic column | Agilent Eclipse C18, 100×4.6 mm, 3.5 µm | |
| Mobile phase | A: 0.05% TFA in H₂O | |
| | B: ACN | |
| | Time (min) | %B |
| | 0.0 | 10 |
| Gradient | 10.0 | 90 |
| | 10.5 | 90 |
| | 15.0 | 10 |
| Run time | 15.0 min | |
| flow rate | 1.0 mL/min | |
| Injection volume | 2/5/10 µL | |
| Wavelength of detection | UV at 254 nm | |
| Column temperature | 25 °C | |
| Automatic injection temperature | RT | |
| Diluted solvent | ACN /H₂O=1:1 (v/v) | |

### Chiral purity test analysis conditions

| Instrument | Agilent 1100 DVD detector | |
|---|---|---|
| Chromatographic column | Chiralcel OD-H, 250×4.6 mm, 5 µm | |
| Mobile phase | A: Heptane/EtOH=95:5 (0.1%DEA) | |
| | Time (min) | %A |
| Gradient | 0.0 | 100 |
| | 35.0 | 100 |
| Run time | 35.0 min | |
| flow rate | 1.0 mL/min | |
| Injection volume | 5 µL | |
| Wavelength of detection | UV at 254 nm | |
| Column temperature | 40 °C | |
| Automatic injection | RT | |
| temperature | | |
| Diluted solvent | EtOH | |

The abbreviation of the solvent and the corresponding name in this specification are shown in the following table, and all solvents can be commercially available.

### Comparison table of solvent names and abbreviations used in experiments

| Abbreviation | Name | Abbreviation | Name |
|---|---|---|---|
| MeOH | Methanol | THF | Tetrahydrofuran |
| EtOH | Ethanol | Cyclohexane | Cyclohexane |
| IPA acetone | Isopropyl alcohol Acetone | ACN DCM | Acetonitrile Methylene chloride |
| MIBK | Methyl isobutyl ketone | toluene | Toluene |
| EtOAc | Ethyl acetate | n-heptane | n-heptane |
| IPAc | Isopropyl acetate | NMP | N-methylpyrrolidone |
| MTBE | Methyl tert-butyl ether | H₂O | Water |

### Example 1: Preparation of Fumarate Crystal Form of the Compound of Formula B-1

### 1.1 Preparation of Fumarate Crystal Form A of Compound of Formula B-1

a. 100 mg of compound of Formula B-1 and 12.5 mg of fumaric acid (molar ratio of 0.5: 1, acid/base) were used as raw materials, and the mixture was suspended and stirred at room temperature in Acetone/n-heptane (1: 4, v/v) for 18 hours in the dark, centrifuged to obtain a solid, and dried at 50°C for 24 hours.

The XRPD characterization results were shown in FIG.1 and Table 1-1.TGA/ DSC results (FIG.2, FIG.3) show that the weight loss is 1.4% when heated to 150°C and there are two endothermic peaks at 127.3°C and 146.1°C (peak temperature). The ¹H NMR result (FIG.4) shows that the molar ratio of fumaric acid to the compound of Formula B-1 in fumarate crystal form A sample is 0.5: 1, and no solvent residue is found. In view of the small TGA weightlessness of the fumarate crystal form A and no other DSC signals observed before melting, it is determined that it is anhydrous crystal form.

**Table1-1 XRPD diffraction peak data of fumarate crystal form A**

| No. | 2θ (°) | Relative intensity (%) | No. | 2θ (°) | Relative intensity (%) |
|---|---|---|---|---|---|
| 1 | 6.01 | 99.96 | 10 | 17.59 | 35.64 |
| 2 | 6.49 | 52.22 | 11 | 18.14 | 26.21 |
| 3 | 9.90 | 32.32 | 12 | 19.00 | 59.15 |
| 4 | 12.21 | 46.41 | 13 | 19.57 | 35.69 |
| 5 | 12.93 | 68.16 | 14 | 20.66 | 41.58 |
| 6 | 13.27 | 62.04 | 15 | 23.28 | 25.69 |
| 7 | 13.65 | 37.63 | 16 | 25.53 | 13.20 |
| 8 | 15.43 | 49.32 | 17 | 28.26 | 18.19 |
| 9 | 16.17 | 100.00 | | | |

Other preparation examples of the fumarate crystal form A include:
b. 30 mg of compound of formula B-1 and fumaric acid (molar ratio of 0.5: 1, acid/base) were used as raw materials, stirred at room temperature in Acetone/n-heptane (1: 4, V/V) for 3 days, and stirred at 5°C for 3 days to obtain the crystal;
c. 1.1 g of compound of formula B-1 and fumaric acid (the molar ratio of acid-base feeding was 0.5: 1) was suspended and stirred for 5 days in acetone/n-heptane (1: 4, V/V) to obtain the crystal;
d. 50 mg of the compound of formula B-1 and fumaric acid (molar ratio of 0.5: 1, acid/base) were stirred at room temperature in acetone/n-heptane (1: 4, v/v) in the dark at room temperature for 2 days, and dried under vacuum at room temperature for 18 hours obtain the crystal.

### 1.2 Preparation of Fumarate Crystal Form B of the Compound of Formula B-1

a. 28.7 mg of compound of formula B-1 and 3.8 mg of fumaric acid (molar ratio of about 0.5: 1, acid/base) were used as raw materials, the mixture was suspended and stirred in 1mL EtOAc/Cyclohexane (1: 4, v/v) in the dark for 2 days at room temperature, after centrifugation, the product was vacuum dried at room temperature for 22 hours to obtain the fumarate crystal form B..

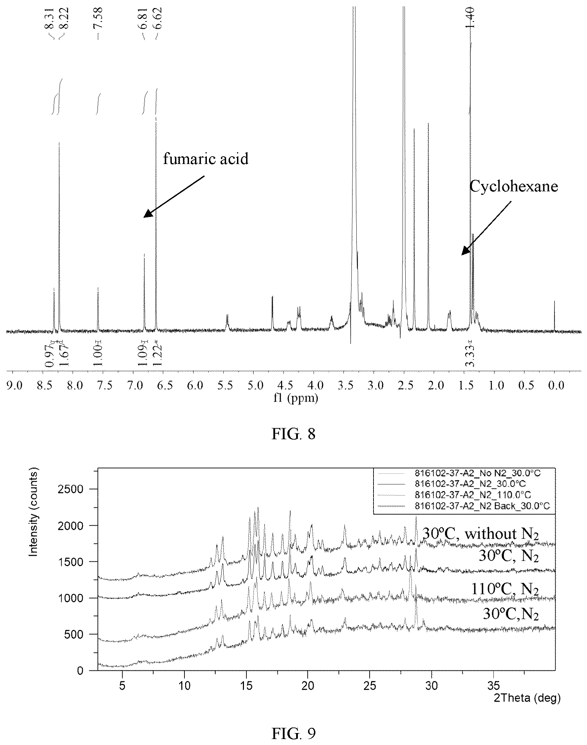

The XRPD characterization results were shown in FIG.5 and Table 1-2. TGA/ DSC results (FIG.6, FIG.7) show that the weight loss is 9.0% when heated to 150°C and there is one endothermic peak at 130.3°C (peak temperature). The ¹H NMR result (FIG.8) shows that the molar ratio of fumaric acid to the compound of formula B-1 in fumarate crystal form B sample is 0.6: 1, and cyclohexane solvent residues are found. The sample is subjected to variable temperature XRPD detection, and the result (FIG.9) shows that the crystal form is unchanged (the partial diffraction peak offset at 110°C is presumed to be caused by lattice expansion at high temperature) when fumarate crystal form B is heated to 110°C under a nitrogen atmosphere and cooled to 30°C, therefore it is determined that it is anhydrous crystal form.

**Table 1-2. XRPD diffraction peak data of fumarate crystal form B**

| No. | 2θ (°) | Relative intensity (%) | No. | 2θ (°) | Relative intensity (%) |
|---|---|---|---|---|---|
| 1 | 12.22 | 15.95 | 11 | 19.06 | 33.18 |
| 2 | 12.73 | 42.46 | 12 | 20.37 | 39.05 |
| 3 | 13.19 | 52.46 | 13 | 20.93 | 14.32 |
| 4 | 15.36 | 73.84 | 14 | 22.85 | 36.88 |
| 5 | 15.78 | 90.75 | 15 | 25.87 | 19.04 |
| 6 | 16.04 | 100.00 | 16 | 26.83 | 42.19 |
| 7 | 16.56 | 64.58 | 17 | 27.96 | 27.22 |
| 8 | 17.21 | 40.05 | 18 | 28.82 | 86.12 |
| 9 | 18.03 | 41.17 | 19 | 29.42 | 34.35 |
| 10 | 18.62 | 87.07 | | | |

Other preparation examples of fumarate crystal form B include:
b. 30 mg of compound of formula B-1 and fumaric acid (molar ratio of 0.5: 1, acid/base) were used as raw materials, stirred at room temperature in EtOAc/Cyclohexane (1: 4, V/V) for 3 days, and dried at room temperature for 2 days to obtain the crystal.

### 1.3 Preparation of Fumarate Crystal Form C of the compound of Formula B-1

a. Approximately 15 mg of fumarate crystal Form A of the compound of formula B-1 was weighed into an HPLC vial, and 0.5 mL of ACN was added. The resulting turbid solution was magnetically stirred (1000 rpm) at room temperature in the dark for 5 days, and then centrifuged (10000 rpm, 2 min) to collect the solid and cooled to dry at room temperature.

The XRPD characterization results are shown in FIG.10 and Table 1-3. TGA/DSC results (FIG.11, FIG.12) show the weight loss is 1.7% when heated to 150°C and there is one endothermic peak at 144.6°C (peak temperature). The ¹H NMR result (FIG.13) shows that the molar ratio of fumaric acid to formula B-1 in the fumarate crystal form C sample is 0.5: 1, and no solvent residue is found. In view of the small TGA weightlessness of the fumarate crystal form C and no other DSC signals observed before melting, it is determined that it is anhydrous crystal form.

**Table 1-3. XRPD diffraction peak data of fumarate crystal form C**

| No. | 2θ (°) | Relative intensity (%) | No. | 2θ (°) | Relative intensity (%) |
|---|---|---|---|---|---|
| 1 | 5.03 | 24.28 | 8 | 17.80 | 45.84 |
| 2 | 10.06 | 30.69 | 9 | 19.41 | 90.07 |
| 3 | 11.04 | 44.96 | 10 | 20.22 | 21.99 |
| 4 | 12.85 | 18.17 | 11 | 21.74 | 51.09 |
| 5 | 15.11 | 100.00 | 12 | 25.15 | 7.65 |
| 6 | 16.06 | 22.69 | 13 | 27.14 | 32.69 |
| 7 | 17.13 | 54.77 | | | |

Other preparation examples of fumarate crystal form C include:
b. 30.2 mg of the compound of formula B-1 was weighed and 1.0 mL ACN was added to dissolve to obtain an alkali solution. 3.8 mg of fumaric acid was weighed, and 0.5 mL ACN was added to dissolve the fumaric acid to obtain an acid solution. The alkali liquor was added dropwise to the acid liquor. The obtained suspension was suspended and stirred at room temperature in the dark for 10 hours, transferred to 5°C, suspended and stirred for 15 hours avoiding light, and centrifuged and separated. The solid was dried under vacuum at room temperature overnight.

### 1.4 Preparation of Fumarate Crystal Form D of the Compound of Formula B-1

a. About 15 mg of fumarate crystal form A of the compound of formula B-1was weighed into an HPLC vial, and 0.5 mL of MTBE was added to obtain a turbid solution. The solution was placed in the dark at room temperature with magnetic stirring (1000 rpm) for 6 days, and then centrifuged (10000 rpm, 2 min) to collect the solid and air-dry at room temperature.

The XRPD characterization results are shown in FIG.14 and Table 1-4. TGA/ DSC results ( FIG.15, FIG.16) show that weight loss is 8.9% when heated to 150°C and there is one endothermic peak at 124.7°C (peak temperature). The ¹H NMR result (FIG.17) show that the molar ratio of fumaric acid to free base in the fumarate crystal form D sample was 0.5:1, the molar ratio of residual solvent MTBE to free base is 0.4: 1, and the corresponding weight loss is 6.3%.

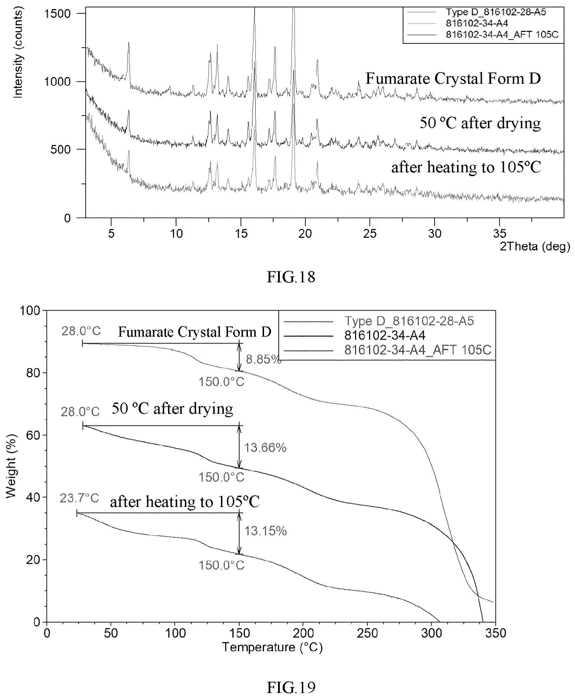

To study the TGA weight loss of fumarate crystal form D, the sample was vacuum dried and heated at 50 °C. The XRPD result (FIG.18) shows that the fumarate crystal form D does not change its crystal form after vacuum drying at 50°C for 9 hours, and the dried sample does not change its crystal form after being heated to 105°C and cooled to room temperature. The TGA result of the sample after drying and heating (FIG.19) shows that the weight loss of the sample is increased, and it is presumed that the heated sample absorbs water in the air. ¹ H NMR (FIG.20) shows that the residual solvent MTBE content remains substantially unchanged after the sample is heated.

**Table 1-4. XRPD diffraction peak data of fumarate crystal form D**

| No. | 2θ (°) | Relative intensity (%) | No. | 2θ (°) | Relative intensity (%) |
|---|---|---|---|---|---|
| 1 | 6.34 | 29.23 | 10 | 19.10 | 100.00 |
| 2 | 9.39 | 3.07 | 11 | 20.45 | 8.25 |
| 3 | 11.31 | 6.41 | 12 | 20.93 | 28.88 |
| 4 | 12.66 | 30.44 | 13 | 21.98 | 5.27 |
| 5 | 13.18 | 38.03 | 14 | 24.15 | 9.44 |
| 6 | 14.04 | 13.49 | 15 | 25.61 | 8.16 |
| 7 | 16.07 | 70.29 | 16 | 25.98 | 8.58 |
| 8 | 17.21 | 12.82 | 17 | 26.95 | 6.94 |
| 9 | 17.63 | 37.74 | 18 | 28.61 | 7.45 |

Other preparation examples of fumarate crystal form D include:
b. About 15 mg of fumarate crystal form A of the compound of formula B-1 was weighed into an HPLC vial, and 0.5 mL NMP/toluene (1: 9, v/v) was added to obtain a turbid solution, the turbid solution was placed in the dark at 5°C with magnetic stirring (1000 rpm) for 5 days, then the solid was collected by centrifugation (10000 rpm, 2 min), and dried at room temperature.

### 2.5 Preparation of Fumarate Crystal Form E of the Compound of Formula B-1

About 15 mg of fumarate crystal form A of the compound of formula B-1 was weighed into an HPLC vial, and 0.5 mL IPA/n-heptane (1:4) was added, after magnetic stirring (1000 rpm) for 5 days in dark at room temperature, then the solid was collected by centrifugation and dried at room temperature.

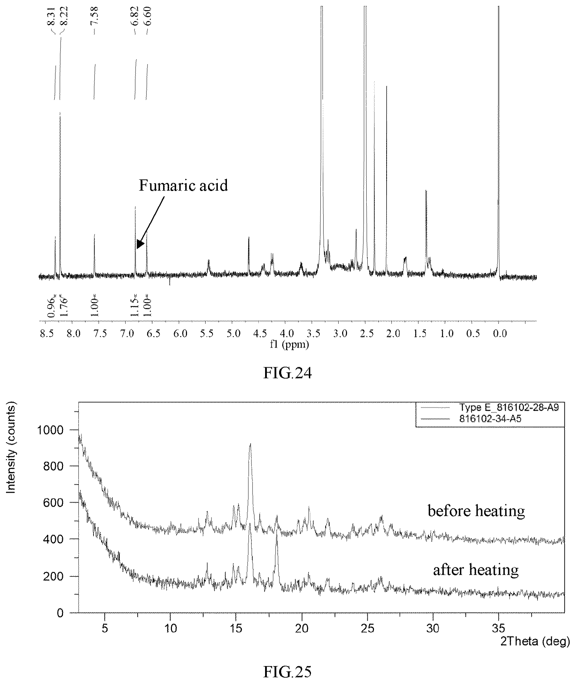

The XRPD characterization results are shown in FIG.21 and Table 1-5. TGA/ DSC results (FIG.22, FIG.23) show that the weight loss is 5.5% at 150°C and there is one endothermic peak at 143.3°C (peak temperature). ¹H NMR results (FIG.24) show that the molar ratio of fumaric acid to formula B-1 in the fumarate crystal form E sample is 0.5:1, and no residual solvent is detected.

After the sample was dried under vacuum at 50°C for 9 hours, the XRPD result (FIG.25) showed the crystal form unchanged. Since the NMR result show that the fumarate crystal form E does not contain residual solvent, TGA weight loss is large and the crystal form is unchanged after vacuum drying at 50°C, it is a hydrate or anhydrous crystal form that is hygroscopic (surface water adsorption).

**Table 1-5 XRPD diffraction peak data of fumarate crystal form E**

| No. | 2θ (°) | Relative intensity (%) | No. | 2θ (°) | Relative intensity (%) |
|---|---|---|---|---|---|
| 1 | 12.80 | 23.28 | 6 | 19.74 | 16.60 |
| 2 | 15.18 | 32.47 | 7 | 20.54 | 33.29 |
| 3 | 16.01 | 100.00 | 8 | 21.96 | 20.71 |
| 4 | 16.82 | 18.41 | 9 | 23.90 | 10.63 |
| 5 | 18.06 | 17.23 | 10 | 25.99 | 20.08 |

### 2.6 Preparation of Fumarate Crystal Form F of the Compound of Formula B-1

a. 500.4 mg of the compound of formula B-1 and 62.6 mg of fumaric acid (molar ratio of 0.5: 1, acid/base) were suspended and stirred at room temperature in 10 mL acetone/n-heptane (1: 4, v/v) in the dark for 2 days, then centrifuged and separated, and then dried under vacuum at room temperature for 22 hours.

The XRPD characterization results are shown in FIG.26 and Table 1-6. TGA/DSC results (FIG.27, FIG.28) show that the weight loss is 2.7% when heated to 150°C and there is one endothermic peak at 158.8°C (peak temperature). The ¹H NMR result (FIG.29 shows that the molar ratio of fumaric acid to formula B-1 in fumarate crystal form F sample is 0.5:1, and no residual solvent is detected. The sample was subjected to variable temperature XRPD detection, and the result (FIG.30) showed that the crystal form was unchanged (the partial diffraction peak offset at 140°C was presumed to be caused by lattice expansion at high temperature) when the fumarate crystal form F was heated to 140°C under nitrogen atmosphere and cooled to 30°C, and therefore it was determined that it was anhydrous crystal form.

**Table1-6. XRPD diffraction peak data of fumarate crystal form F**

| No. | 2θ (°) | Relative intensity (%) | No. | 2θ (°) | Relative intensity (%) |
|---|---|---|---|---|---|
| 1 | 5.54 | 18.41 | 11 | 20.95 | 32.70 |
| 2 | 9.57 | 4.06 | 12 | 21.49 | 14.24 |
| 3 | 11.06 | 100.00 | 13 | 22.38 | 38.21 |
| 4 | 14.46 | 15.47 | 14 | 23.31 | 9.78 |
| 5 | 15.47 | 14.44 | 15 | 25.03 | 25.41 |
| 6 | 16.60 | 55.55 | 16 | 27.44 | 7.39 |
| 7 | 16.90 | 28.06 | 17 | 28.86 | 8.10 |
| 8 | 17.49 | 15.01 | 18 | 29.89 | 7.64 |
| 9 | 18.64 | 33.33 | 19 | 30.45 | 7.92 |
| 10 | 19.16 | 6.43 | | | |

Other preparation examples of the fumarate crystal form F include:
b. Obtained by suspension stirring of fumarate crystal form A of the compound of formula B-1

About 15 mg of fumarate crystal form A of the compound of formula B-1 was weighed to HPLC glass vial, and 0.5 mL of the solvent listed in Table 2-7 was added respectively. The turbid liquid obtained was placed under magnetic stirring (1000 rpm) at different temperatures for about 5 days, then centrifuged (10000 rpm, 2 min) to collect the solid and perform XRPD testing. The test results are shown in Table 1-7.

**Table 1-7 Results of Room Temperature Suspension Stirring Test**

| N O. | Solv ent | T | Tim e | Result |
|---|---|---|---|---|
| 1 | MTB E | 50°C | 5d | Form F |
| 2 | MIB K | 5°C | 5d | Form F |

### c. Obtained by the compound of formula B-1 reacting with fumaric acid

The compounds of formula B-1 shown in Table 2-8, along with the corresponding proportions of fumaric acid, were suspended and stirred at room temperature in the indicated solvents in the dark for 4 days, centrifuged, and then dried under vacuum at room temperature to prepare the product. The test results are shown in Table 1-8.

**Table 1-8. Preparation of Fumarate Crystal Form F of the Compound of Formula B-1**

| Material | Amount (mg) | ratio | Solvent(v/v) | Operation | Time | Result |
|---|---|---|---|---|---|---|
| Free base +acid | 10 | 0.5:1 | ACN | room temperature, Light Shielding, | 4d | form F |
| Free base +acid | 50 | 0.5:1 | MTBE | Suspension Stirring room temperature, Light Shielding, Suspension | 4d | form F |
| Free base +acid | 50 | 0.5:1 | IPA/n-heptane (1:4) | Stirring room temperature, Light Shielding, Suspension | 4d | form F |
| Free base +acid | 50 | 0.5:1 | EtOH/H₂O (1:9) | Stirring 50°C, Light Shielding, Suspension | 4d | form F |
| Free base +acid | 50 | 0.5:1 | IPA/n-heptane (1:4) | Stirring 50°C, Light Shielding, Suspension Stirring | 4d | form F |

### d. Obtained by suspension stirring of fumarate crystal form A, B, and C of the compound of formula B-1

The crystal form F of fumarate was weighed into HPLC vials, and corresponding solvent was added, then suspended and stirred at room temperature/50°C for 4 or 1.5 hours, then filtered into HPLC vials containing each crystal form by a 0.45 µm pore size PTFE filter membrane and then suspended and stirred at room temperature/50°C, respectively. XRPD was tested on each sample, and the results were summarized in Table 1-9.

**Table 1-9 Preparation of Fumarate Crystal Form F of the Compound of Formula B-1**

| Start Form | solvent | T | Tim e | Solid Form |
|---|---|---|---|---|
| fumarate crystal form A+B+F | Acetone | Room temperature | 4 d | form F |
| | ACN | Room temperature | 6 d | form F+C* |
| | Acetone | 50 °C | 4 d | form F |
| | ACN | 50 °C | 6 d | form F+C* |
| fumarate crystal form F+C | MTBE | Room temperature | 17h | form F |
| | IPAc | Room temperature | 17h | form F |
| | MTBE | 50 °C | 17h | form F |
| | IPAc | 50 °C | 17h | form F |

| | | | | |
|---|---|---|---|---|
| *: It is speculated that due to the influence of the solvent (crystal form C was obtained by solution crystallization in ACN), and the sample did not completely transform into crystal form F after stirring for 6 days. | | | | |

### 2.7 Preparation of Fumarate Crystal Form G of the Compound of Formula B-1

a. 15 mg of fumarate crystal form A of the compound of formula B-1 was weighed into an HPLC glass vial, 0.5 mL EtOH/H₂O (1:9) was added, and the obtained suspension was magnetic stirred (1000 rpm) at 50°C in the dark for 5 days, and then centrifuged (10000 rpm, 2 min) to collect the solid.

The XRPD characterization results are shown in FIG.31 and Table 1-10. TGA/DSC results (FIG. 32, FIG. 33) show that the weight loss is 4.0% when heated to 150°C and there are two endothermic peaks at 70.8°C and 170.1°C (peak temperature). The ¹H NMR result (FIG.34) shows that the molar ratio of fumaric acid to free base in the fumarate crystal form G sample is 0.3:1, and the molar ratio of the residual solvent EtOH to the free base is 0.3: 1.

**Table 1-10. XRPD diffraction peak data of fumarate crystal form G**

| No. | 2θ (°) | Relative intensity (%) |
|---|---|---|
| 1 | 15.60 | 66.35 |
| 2 | 17.69 | 100.00 |
| 3 | 20.63 | 33.24 |

Other preparation examples of the fumarate crystal form G include:
b. About 15 mg of fumarate crystal form A of the compound of formula B-1 was weighed into an HPLC glass vial, and 0.5 mL NMP/H₂O (1: 9) was added, and the obtained suspension was magnetic stirred (1000 rpm) at 50°C for 5 days, and then centrifuged (10000 rpm, 2 min) to collect the solid.

### 2.8 Preparation of Fumarate Crystal Form H of the Compound of Formula B-1

About 15 mg of fumarate crystal form A of the compound of formula B-1 was weighed into an HPLC glass vial and 0.5 mL IPA/n-Heptane (1: 9, v/v) was added, and the obtained suspension was magnetic stirred (1000 rpm) at 50°C in the dark for 5 days, and then centrifuged (10000 rpm, 2 min) to collect the solid.

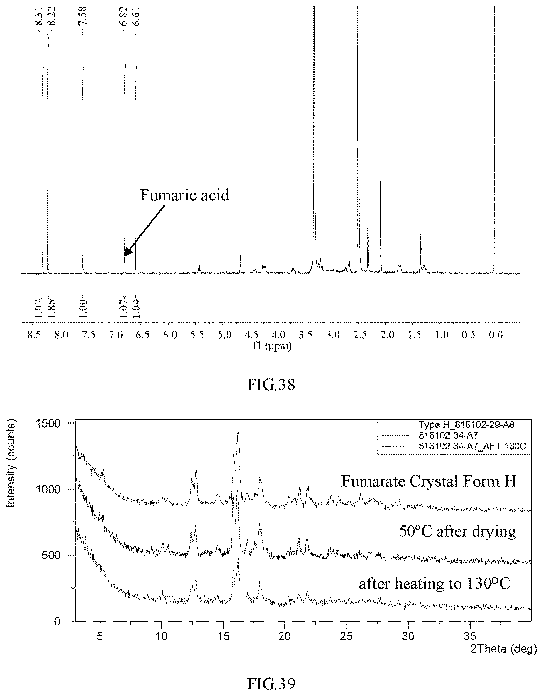

The XRPD characterization results are shown in FIG.35 and Table 1-11. TGA/DSC results (FIG.36, FIG.37) show that the weight loss is 4.4% at 150°C and there is one endothermic peak at 156.0°C (peak temperature). ¹H NMR results (FIG.38) show that the molar ratio of fumaric acid to free base in fumarate crystal form H sample is 0.5:1, and no residual solvent is detected.

**Table 1-11 XRPD diffraction peak data of fumarate crystal form H**

| No. | 2θ (°) | Rel. Int. [%] | No. | 2θ (°) | Rel. Int. [%] |
|---|---|---|---|---|---|
| 1 | 5.23 | 11.30 | 9 | 20.33 | 10.61 |
| 2 | 12.44 | 33.48 | 10 | 21.19 | 23.93 |
| 3 | 12.81 | 45.21 | 11 | 21.84 | 25.56 |
| 4 | 14.54 | 13.97 | 12 | 23.72 | 12.36 |
| 5 | 15.82 | 64.04 | 13 | 26.12 | 13.59 |
| 6 | 16.18 | 100.00 | 14 | 27.39 | 8.21 |
| 7 | 16.93 | 16.95 | 15 | 29.25 | 9.47 |
| 8 | 17.99 | 36.65 | | | |

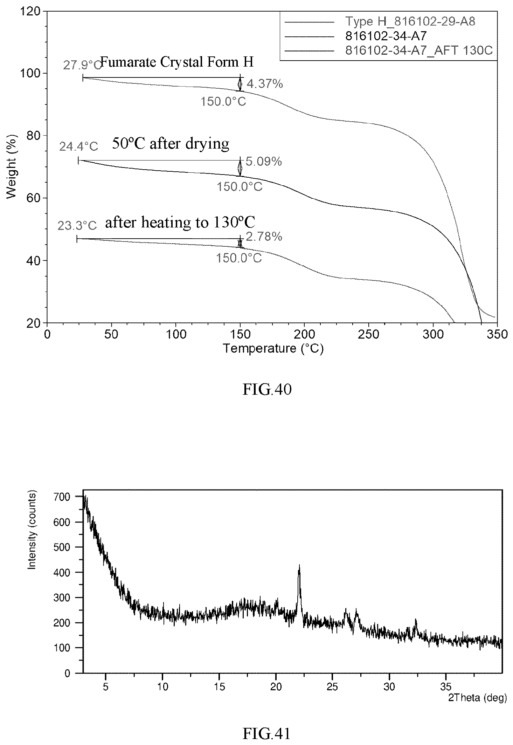

To study TGA weight of fumarate crystal form H, the sample was vacuum dried and heated at 50°C. The XRPD results (FIG.39) show that the crystal form of fumarate crystal form H remains unchanged after vacuum drying at 50°C for 9 hours, and the crystal form of dried sample remains unchanged after heating to 130°C and cooling to room temperature. The TGA result of the sample after drying and heating (FIG.40) shows that the weight loss decreases after heating. Since the NMR result shows there isn't residual solvent in fumarate crystal form H, and TGA weightlessness is large and the crystal form remain unchanged after heating, the fumarate crystal form H is hydrate or anhydrous crystal form that is hygroscopic (surface water adsorption).

### Example 2: Preparation of Succinate Crystal Form of the Compound of Formula B-1

### 2.1 Preparation of succinate crystal Form A of the Compound of Formula B-1

About 15 mg of free base material was stirred with equimolar acid at 5°C in 0.5 ml methyl tert-butyl ether for 3 days, stirred at -20°C for 2 days, and then volatilized and dried at room temperature to collect solids.

The XRPD characterization results are shown in FIG. 41 and Table 2-1.

**Table 2-1 XRPD diffraction peak data of succinate crystal form A**

| No. | 2θ (°) | Relative intensity (%) |
|---|---|---|
| 1 | 22.06 | 100.00 |
| 2 | 27.17 | 31.38 |

### 2.2 Preparation of succinate crystal form B of the compound of formula B-1

About 15 mg of free base material was suspended and stirred with equimolar acid at room temperature for 5 days in 0.5 ml mixed solvent of acetone and n-heptane for 5 days, and then centrifuged to collect solids.

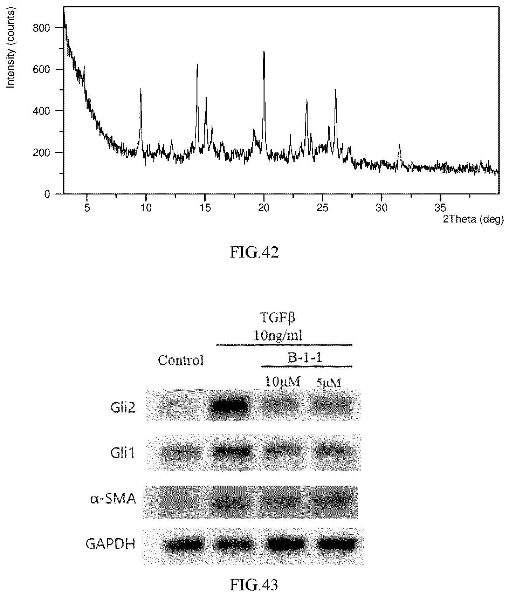

The XRPD characterization results are shown in FIG. 42 and Table 2-2.

**Table 2-2 XRPD diffraction peak data of succinate crystal form B**

| No. | 2θ (°) | Relative intensity (%) | No. | 2θ (°) | Relative intensity (%) |
|---|---|---|---|---|---|
| 1 | 9.54 | 55.51 | 7 | 19.18 | 28.52 |
| 2 | 12.18 | 14.05 | 8 | 22.27 | 22.69 |
| 3 | 14.37 | 86.97 | 9 | 23.65 | 55.57 |
| 4 | 15.12 | 53.48 | 10 | 24.04 | 21.76 |
| 5 | 15.62 | 26.19 | 11 | 25.54 | 32.16 |
| 6 | 16.50 | 13.92 | 12 | 27.26 | 12.11 |

### Example 3: Ability of the fumarate crystal form F of formula B-1 (Formula B-1-1) to degrade GLi 1, GLi 2, and α-SMA proteins induced by TGF-β

### 1. Experimental material and reagent

### 1.1 Preparation of the compound of formula B-1-1:

1) The preparation reference patent WO2018/006756 A1 of formula B-1 is described in Example 1.
2) The preparation of fumarate crystal form F (compound B-1-1) of formula B -1 is as follows:
3) 83 g (98.9% purity) of the compound of formula B-1 was dissolved in 264 g of acetone, stirred at room temperature for 30 minutes, the insoluble substance was removed by suction filtration, and the filtrate was collected;
4) under the condition of 0-25°C, adding 10.38 g (0.5 eq) of fumaric acid to the filtrate, and stirring for 12 hours;
5) 914 g of n-heptane was added and stirred at 0 -25°C for 2 h;
6) The filter cake was washed with 200 g of n-heptane, and air- dried at 50 -55°C to a constant weight to obtain 91 g of the fumarate crystal form F (compound B-1-1). Purity 99%, yield 97.6%.

¹H NMR (400 MHz, DMSO) δ13.13(br s, 1H),δ 8.31 (s, 1H), δ8.21-8.22 (d, 2H), δ7.56(s, 1H), δ6.81(s, 1H), δ6.63(s, 1H), δ5.42-5.43 (m, 1H),δ4.68(br s, 1H), δ4.40-4.42 (m , 1H),δ4.23-4.26 (m, 2H),δ3.69-3.70 (m, 1H), δ3.34(m, 1H), δ3.16-3.22 (m, 2H),δ2.68-2.76 (m , 2H), δ2.32(s, 3H), δ2.09(s, 3H), δ1.73-1.75 (m,2H),δ1.34-1.35 (d, 3H),δ1.22-1.30 (m , 2H)_{∘}

The ¹H NMR results show that the molar ratio of compound B-1 to fumaric acid in compound B-1-1 is 2: 1.

In addition to compound B-1-1, the experimental materials and reagents used in the present application are commercially available.

### 1.2 Cell Lines and Sources

Human lung carcinoma cells (A549 cell line) were purchased from Shanghai Cell Bank of Chinese Academy of Sciences.

### 1.3 Cell Culture Conditions

The cells were cultured in RPMI (Roswell Park Memorial Institute)-1640 medium supplemented with 10% FBS (Fetal Bovine Serum) and 1% penicillin-streptomycin. The cell culture conditions were 5% CO₂ and 37% humidity. Upon reaching approximately 80% confluence, the cells were subcultured at a 1:3 ratio.

### 2. Cell Drug Treatment and Protein Extraction

### 2.1 Cell Treatment

A549 cells in the exponential growth phase were digested and plated into 6-well plates at 1×10⁶ cells per well. After one day of growth, TGF-β (transforming growth factor-β) and different concentrations of compound B-1-1 were added to culture the cells. Protein was extracted 24 hours after drug treatment.

### 2.2 Cellular Protein Extraction

After removing the culture medium from the 6-well plates, the cells were washed once with PBS (phosphate-buffered saline) and then trypsinized. The cells were collected in 1.5 mL microcentrifuge tubes by centrifugation. Add 100 µL of RIPA lysis buffer (Radio Immunoprecipitation Assay Lysis buffer) (containing 100 µM PMSF (Phenylmethylsulfonyl fluoride)) to each tube. The mixture was vortexed thoroughly and incubated on ice for 30 min. Subsequently, the lysates were centrifuged at 12,000 rpm for 20 min at 4°C. The supernatant was collected for Western blotting (WB) assay, and the samples can be stored at -80°C.

### 2.3 Protein Concentration Measurement

The protein concentration was determined using a BCA Protein Assay Kit. BSA standard solutions and the supernatant from section 2.2 (the supernatant can be diluted before detection) were prepared according to Table 3-1. The samples were loaded into a 96-well plate, and each well was supplemented with PBS to a total volume of 20 µL. Then, 200 µL of BCA working solution (prepared according to the kit instructions) was added to each well. After thoroughly mixing, the plate was incubated at 60°C for 10 min, and the absorbance was measured at 562 nm. Record the readings and plot a standard curve based on the standard concentration gradient. The protein concentration of the samples was calculated by substituting the sample absorbance values into the standard curve.

**Table 3-1 Preparation of Protein Quantification Standards**

| No. | 0 | 1 | 2 | 3 | 4 | 5 | 6 | 7 | 8 |
|---|---|---|---|---|---|---|---|---|---|
| | 1 mg/mL BSA Standard Solution (µL) | | | | | | | 5 mg/mL BSA Standard Solution (µL) | |
| BSA Standard Solution (µL) | 0 | 0.5 | 2.5 | 5.0 | 10 | 15 | 20 | 6 | 8 |
| PBS Solution (µL) | 20 | 19.5 | 17.5 | 15 | 10 | 5 | 0 | 14 | 12 |
| Final BSA Concentration (µg/mL) | 0 | 25 | 125 | 250 | 500 | 750 | 1000 | 1500 | 2000 |
| Total Volume (µL) | 20µL | | | | | | | | |

### 3. Standard Procedure for Western Blotting

### 3.1 Protein Denaturation

Take out the RIPA protein lysate, add 5× Loading Buffer (SDS, glycerol, bromophenol blue, TRIS), and denature at 100°C for 5 minutes.

### 3.2 Sample Loading and Electrophoresis

Use 10% 15-well pre-prepared gel and corresponding electrophoresis buffer. Load the same mass of protein samples and protein ladder into each well, then electrophoresis at 200V for 30 minutes.

### 3.3 Blocking

Remove the gel and cut off the excess part. Transfer it onto a PVDF membrane (polyvinylidene difluoride) using the wet transfer method (the PVDF membrane needs to be activated with methanol for 1 minute before use). Transfer was performed at 300 mA for 2 hours. Due to heat generation during transfer, an ice pack was used to maintain cooling.

The transferred PVDF membrane was placed in 5% (w/w) skim milk and shaken at room temperature for 1 h to block.

### 3.4 Primary Antibody Incubation

The PVDF membranes were cut according to the molecular weight markers and separately incubated with primary antibodies against GLil, GLi2, α-SMA, and GAPDH. The antibodies were diluted at a 1:1000 ratio in TBST buffer (a solution of Tris, KCl, and NaCl, adjusted to pH 7.4 with HCl, and Tween 20 added). The membranes were incubated at 4°C on a shaker overnight.

### 3.5 Secondary Antibody Incubation

After primary antibody incubation, the PVDF membranes were washed three times with TBST on a shaker, 10 minutes each time. After washing, place the membrane in the Anti-mouse IgG, HRP-linked Antibody or Anti-rabbit IgG, HRP-linked Antibody, incubation chambers at room temperature for 2 hours on the shaker.

### 3.6 Membrane Washing and Chemiluminescent Detection

After incubation with the secondary antibodies, place the membrane in TBST on a shaker and wash 3 times, 10 minutes each time. After washing, protein bands were visualized using enhanced chemiluminescence (ECL), which detects the intensity of chemiluminescent signals.

### 4. Results

According to the above method, the degradation abilities of GLil, GLi2 and α-SMA proteins in A549 cells were tested. The results are shown in Figure 43. As shown in Figure 43, After A549 cells induced by TGF-β, the expression levels of GLil, GLi2, and the pulmonary fibrosis-associated protein α-SMA were significantly upregulated. After 24-hour treatment with compound B-1-1, the results demonstrated that compound B-1-1 effectively degraded TGF-β-induced GLil, GLi2, and α-SMA proteins. These findings indicate that the compound of the present application significantly downregulates the expression of GLil, GLi2, and the fibrosis-related protein α-SMA. It confirms that compound B-1-1 can be used for the treatment of idiopathic pulmonary fibrosis (IPF) by inhibiting the effects of GLil, GLi2 and α-SMA.

### Example 4: Effect of Compound B-1 Fumarate Crystal Form F (the Compound of formula B-1-1) on Bleomycin-induced Unilateral Pulmonary Fibrosis Model in SD Rats

### 1. Experimental Materials

55 SD rats were purchased from Zhejiang Charles River Laboratory Animal Technology Co., Ltd. and housed in the SPF-grade barrier system of KCI Biotechnology (Suzhou) Co., Ltd. The animal use certification number was SYXK (Su) 2017-0041, the temperature, humidity and light control system follows international standards.

### 2. Establishment of Pulmonary Fibrosis Model and Grouping Administration

### 2.1 Establishment of Pulmonary Fibrosis Model

Anesthesia: 1-2.5% isoflurane was used for respiratory anesthesia to maintain deep anesthesia of the animals.

Establishment of unilateral pulmonary fibrosis model in rats:
After the rats were under surgical anesthesia, the experimental model was constructed according to the guidelines for establishing the unilateral pulmonary fibrosis model in KCI. The skin was shaved, locally disinfected, and then a layered incision was made to expose and preserve the trachea. Bleomycin (dose: 3 mg/kg) was administered via intratracheal instillation to induce left lung fibrosis. After confirming no bleeding, the incision was closed layer-by-layer using 2-0 silk surgical sutures. After the surgery, the animals were placed on a 37°C electric heating pad until fully recovered. Normal feeding and drinking were confirmed before returning the animals to standard housing. Starting from the day of modeling, meloxicam (1 mg/kg) was administered subcutaneously once daily for three consecutive days for postoperative analgesia.

### 2.2 Preparation of Test and Reference Compounds

### 2.2.1 Solvent Preparation

Weigh 5g of MC (Methyl cellulose), dissolve it in 1L of water solution according to the preparation requirements, stir until the solution becomes clear and free of flocculent substances, then add 2mL of Tween80 and mix well to obtain 1L of 0.5% MC + 0.2% Tween80 solution for later use.

### 2.2.2 Preparation of Compound B-1-1 Solution

The preparation method of compound B-1-1 refers to Example 3.

60.48 mg of compound B-1-1 was weighed and dissolved in 30 mL of 0.5% MC + 0.2% Tween80 aqueous solution according to the preparation requirements, and then ultrasonicated until the solution was uniform and free of precipitates, 30 mL of 2 mg/mL compound B-1-1 solution was obtained for later use.

120.97 mg of compound B-1-1 was weighed and dissolved in 30 mL of 0.5% MC + 0.2% Tween 80 aqueous solution according to the preparation requirements, and then ultrasonicated until the solution was uniform and free of precipitates, 30 mL of 4 mg/mL compound B-1-1 solution was obtained for subsequent use.

### 2.2.3 Preparation of Nintedanib

303.03 mg of nintedanib (purchased from Shanghai ChemBest Chemical, CL-20210522) was weighed and dissolved in 30 mL of 0.5% MC + 0.2% Tween 80 aqueous solution according to the preparation requirements, and then ultrasonicated until the solution was uniform and free of precipitates. 30 mL of 10 mg/mL nintedanib solution for subsequent use.

### 2.3 Experimental Grouping

Prior to modeling, animals were randomly divided into five groups based on body weight, as follows: Sham model group (normal group) (n=10), model group (n=10), Nintedanib 100 mg/kg, qd group (n=10), Compound B-1-1 20 mg/kg, bid group (n=10), Compound B-1-1 40 mg/kg, bid group (n=10). The administration began on the 8th day after modeling.
Nintedanib: administered orally, once daily for 28 consecutive days;
Compound B-1-1: administered orally, twice daily for 28 consecutive days. Detailed grouping and dosing regimens are shown in Table 4-1.

**Table 4-1 Experimental Grouping and Dosing Regimens**

| Group | Number of Animals | Model | Test Compound | Dose | Route/ Frequency | Dosing Volume | Study Duration |
|---|---|---|---|---|---|---|---|
| 1 | 10 | Sham | Vehicle | NA | p.o., q.d. | 10 mL/kg | 35 days |
| 2 | 10 + 1* | Yes | Vehicle | NA | p.o., q.d. | 10 mL/kg | 35 days |
| 3 | 10 | Yes | Nintedanib | 100 mg/kg | p.o., q.d. | 10 mL/kg | 35 days |
| 4 | 10 | Yes | Compound B-1-1 | 20 mg/kg | p.o., b.i.d. | 10 mL/kg | 35 days |
| 5 | 10 | Yes | Compound | 40 mg/kg | p.o., b.i.d. | 10 mL/kg | 35 days |
| | | | B-1-1 | | | | |

| | | | | | | | |
|---|---|---|---|---|---|---|---|
| Note: *indicates a spare (reserve) animal. | | | | | | | |

Sham refers to the sham model group (normal control), without disease modeling. YES refers to the model group, which underwent disease model induction.

### 3. Evaluation Indicators

### 3.1 Animal Body Weight

Animals were weighed and grouped one day prior to model induction. Subsequently, body weight measurements were taken every two days until the end of the study.

### 3.2 Study Endpoint

After euthanasia, animals were perfused systemically with pre-cooled PBS at 4°C. The lungs were collected, and the left lung lobes were perfused with formalin. Histopathological examinations were performed using HE staining and Masson Trichrome staining. HE staining was used to evaluate the extent of bronchial and small pulmonary artery injury and inflammation, while Masson Trichrome staining assessed the degree of pulmonary fibrosis.

### 3.3 Histopathological Analysis of Left Lung Tissue

The entire left lung was dehydrated and embedded in paraffin. Paraffin sections of the entire left lung were prepared at a thickness of 3 µm. HE-stained sections were used for pathological analysis and scoring of injury and inflammatory changes in terminal bronchioles and accompanying small pulmonary arteries within and surrounding the lesion (Tables 4-2 and 4-3). Masson Trichrome staining was performed on the left lung paraffin sections, and the stained sections were scanned using the Nano Zoomer Digital Pathology scanner (S210) to obtain whole-slide images. Ten areas of 1 mm² each were randomly selected within the lesion area, and semi-quantitative scoring was conducted under a double-blind condition according to the Ashcroft scoring system (Table 4-4).

**Table 4-2 Pathological Evaluation Criteria for Injury and Inflammatory Infiltration of Terminal Bronchiolar**

| Score | Injury of Terminal Bronchiolar Wall | Inflammatory Infiltration in the Terminal Bronchiolar Wall |
|---|---|---|
| 0 | Normal tissue structure. | Normal tissue structure, no observable inflammatory cell infiltration. |
| 1 | Normal tissue structure accompanied by injury in less than 1/2 of the bronchiolar wall, characterized by bronchiolar epithelial damage and regeneration, wall edema, and degeneration or regeneration of the muscularis. | Scattered inflammatory cell infiltration observed in the adventitia, non-focal, with fewer than 10 inflammatory cells. |
| 2 | Normal tissue structure accompanied by injury in more than 1/2 of bronchiolar wall, characterized by bronchiolar epithelial damage and regeneration, wall edema, and degeneration or regeneration of the muscularis. | Scattered and abundant inflammatory cell infiltration in the adventitia, forming one or multiple foci, involving less than 1/2 of the bronchiolar wall. |
| 3 | Normal tissue structure accompanied by injury in more than 1/2 of bronchiolar wall, characterized by bronchiolar epithelial damage and regeneration, wall edema, degeneration or regeneration of muscularis, and granuloma formation or fibrosis in the adventitia. | Diffuse inflammatory cell infiltration in the adventitia involving more than 1/2 of the bronchiolar wall, or infiltration into the mucosa and muscularis. |

**Table 4-3 Pathological Evaluation Criteria for Injury and Inflammatory Infiltration of Small Pulmonary Arteries**

| Score | Injury of Small Pulmonary Arterial Wall | Inflammatory Cell Infiltration in Small Pulmonary Arteries |
|---|---|---|
| 0 | Normal structure of small pulmonary arteries. | Normal structure of small pulmonary arteries. |
| 1 | Partial detachment of endothelial cells. | Scattered inflammatory cell infiltration in the adventitia, non-focal, with fewer than 10 inflammatory cells. |
| 2 | Endothelial cell detachment, degeneration, proliferation, or focal necrosis of the muscularis. | Scattered and abundant inflammatory cell infiltration in the adventitia, forming one or multiple foci, involving less than 1/2 of the adventitial area. |
| 3 | Endothelial cell detachment; degeneration, proliferation, or focal necrosis of muscularis, granuloma formation or fibrosis in the adventitia. | Diffuse inflammatory cell infiltration in the adventitia involving more than 1/2 of the arterial wall area, or infiltration into the mucosa. |

**Table 4-4 Pathological Evaluation Criteria for Pulmonary Fibrosis**

| Fibrosis Grade | Ashcroft Scoring Criteria |
|---|---|
| 0 | Alveolar septa: No fibrotic lesions. |
| | Lung structure: Normal. |
| 1 | Alveolar septa: Isolated mild fibrotic changes (alveolar septa thickened, but less than three times that of normal lung). |
| | Lung structure: Partially enlarged, minimal exudate, no fibrotic material present. |
| 2 | Alveolar septa: Definite fibrotic changes (alveolar septa thickened, more than three times that of normal lung), forming small, non-confluent nodules. |
| | Lung structure: Partially enlarged, minimal exudate, no fibrotic material present. |
| 3 | Alveolar septa: Non-continuous fibrosis visible in nearly all alveolar walls under high-power field (alveolar septa thickened, more than three times that of normal lung). |
| | Lung structure: Partially enlarged, minimal exudate, no fibrotic material present. |
| 4 | Alveolar septa: Still visible. |
| | Lung structure: Presence of isolated fibrotic nodules (≤10% of high-power field). |
| 5 | Alveolar septa: Still visible. |
| | Lung structure: Presence of confluent fibrotic nodules (>10% and ≤50% of high-power field); lung structure severely damaged but partially preserved. |
| 6 | Alveolar septa: Barely visible or nearly absent. |
| | Lung structure: Extensive, continuous fibrotic nodules (>50% of high-power field); lung architectural structure nearly absent. |
| 7 | Alveolar septa: Absent. |
| | Lung structure: Almost completely filled with fibrotic tissue, but 5 or fewer vacuolated structures remain. |
| 8 | Alveolar septa: Absent. |
| | Lung structure: Completely filled with fibrotic tissue under high-power field. |

### 3.4 Data Analysis

Statistical analysis was performed using Prism 6.0 software. Data are expressed as mean ± SEM (standard error). One-way ANOVA was used for the analysis, and Tukey's method was selected for the comparison of differences between groups; when comparing between two groups, a T-test was used for two-tailed detection; when p < 0.05, there was a significant difference between the two groups. In cases where outliers were suspected due to large within-group variability, the Identify Outliers function in GraphPad Prism 6.0 was used to detect and exclude outlier values. For data analysis purposes, all relevant data from any animal excluded from the efficacy evaluation were also omitted from statistical analysis.

### 4. Experimental Results

### 4.1 Clinical Observations During Animal Study

No animal deaths occurred during the entire study period. On the day of bleomycin-induced model establishment, the animals showed symptoms such as poor mental state, reduced water intake and food consumption, and decreased activity. From the first to the fifth day of the model establishment, a downward trend in body weight was observed, followed by gradual recovery to normal levels. Drug administration began on Day 8 after modeling. No drug-related clinical abnormalities were observed throughout the treatment period.

### 4.2 Changes in Body Weight

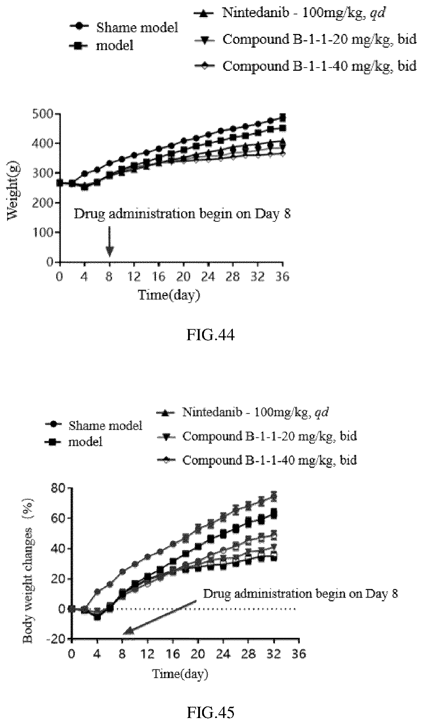

As shown in Figures 44 and 45, body weight changes indicated that during the early stage of the model (Day 1 to Day 5), both the model group and all treatment groups exhibited a decreasing trend in body weight, followed by recovery and stable weight gain. Compared with the model group, animals in Group 5 (Compound B-1-1 40 mg/kg) showed a slower rate of weight gain after administration, and there were no significant differences in the weights of animals in the other treatment groups.

### 4.3 Pathological Evaluation of Injury and Inflammatory Changes in Terminal Bronchioles and Accompanying Small Pulmonary Arteries Within and Surrounding Left Lung Lesions

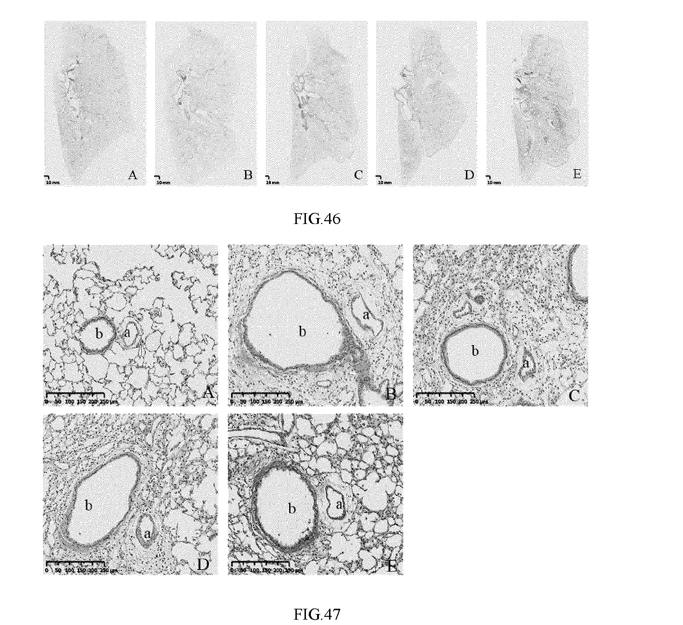

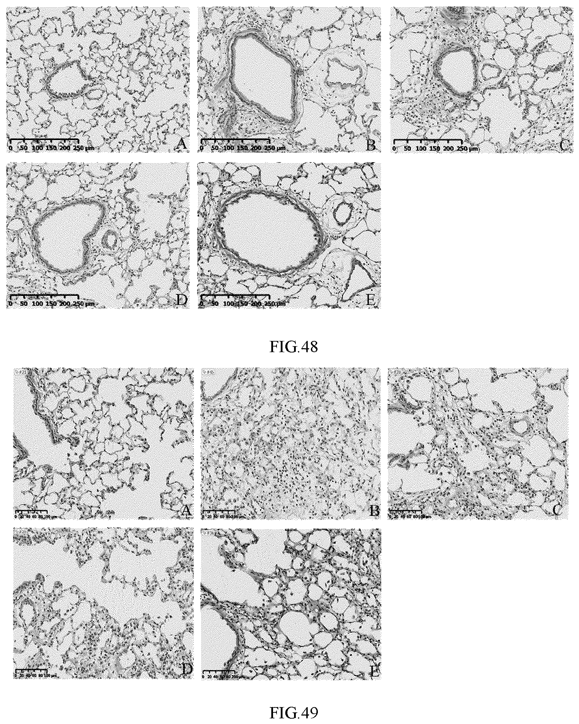

The HE staining of the left lung pathology showed that, compared to the sham group, the model group and all treatment groups exhibited pathological changes characterized by varying degrees of proliferation in the epithelium of bronchioles, terminal bronchioles, and alveolar ducts. Inflammatory cell infiltration was observed in the bronchial walls, with partial wall thickening and granuloma formation in the adventitia. Small pulmonary artery injury was manifested as endothelial cell detachment and inflammatory cell infiltration. Alveolar tissue within lesions displayed epithelial detachment and regeneration, alveolar wall thickening, inflammatory exudate and cell infiltration within alveolar spaces, and partial loss of normal alveolar architecture (Figures 46, 47, 48, 49).

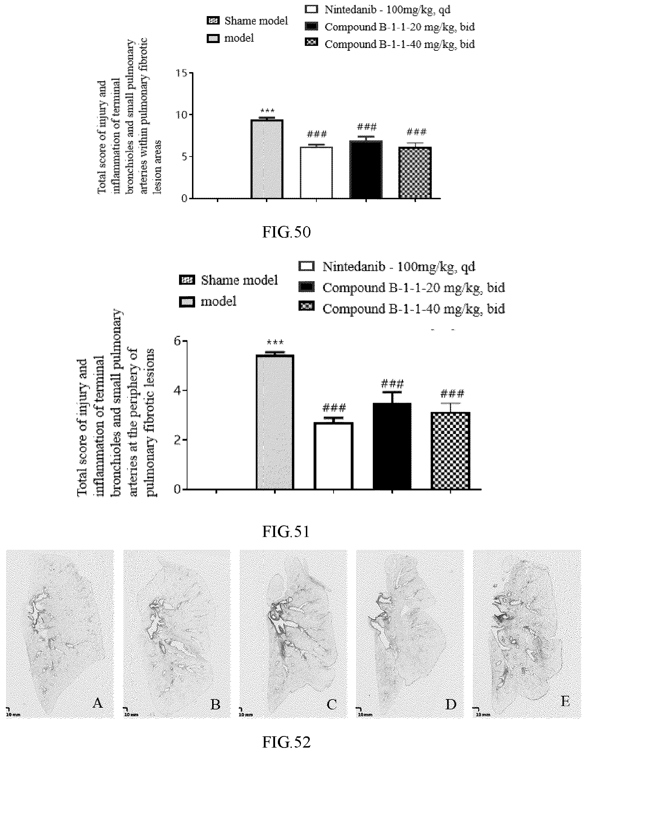

Pathological injury scores of terminal bronchioles and accompanying small pulmonary arteries within fibrotic lesions and at lesion margins were evaluated. Compared with the first group (sham model group), the model group showed significantly increased injury and inflammatory infiltration scores in both terminal bronchioles and small pulmonary artery walls within and at the margins of fibrotic lesions (*p* < 0.001, *p* < 0.001; Figures 50-51). Statistical analysis by one-way ANOVA is indicated as *** p < 0.001 vs. sham group, ^{###} p < 0.001 vs. model group in Figures 50-51.

Compared to the model group, treatment with nintedanib significantly reduced inflammatory cell infiltration and injury severity in terminal bronchioles and small pulmonary arteries within and surrounding fibrotic lesions (*p* < 0.001; Figures 50-51). Both the two different dose of compound B-1-1 significantly decreased inflammatory infiltration and pathological injury in terminal bronchioles and small pulmonary arteries within and at the margins of fibrotic lesions (*p* < 0.001, *p* < 0.001; Figures 50-51) (Table 4-5).

**Table 4-5 Changes in Injury Scores of Terminal Bronchioles and Accompanying Small Pulmonary Arteries Within and at the Margins of Left Lung Fibrotic Lesions (Mean ± SEM)**

| Group | Within Lesion | At Margin Lesion |
|---|---|---|
| Sham Group | 0.00 ± 0.00 | 0.00 ± 0.00 |
| Model Group | 9.44 ± 0.22 *** | 5.44 ± 0.12 *** |
| Nintedanib 100 mg/kg, q.d. | 6.24 ± 0.21 ^{###} | 2.72 ± 0.17 ^{###} |
| Compound B-1-1 20 mg/kg, b.i.d. | 6.96 ± 0.46 ^{###} | 3.50 ± 0.43 ^{###} |
| Compound B-1-1 40 mg/kg, b.i.d. | 6.20 ± 0.47 ^{###} | 3.14 ± 0.35 ^{###} |

| | | |
|---|---|---|
| Note: One-way ANOVA: *** p < 0.001 vs. Sham group; ### p < 0.001 vs. Model group. | | |

### 4.4 Changes in Left Lung Fibrotic Lesions and Pathological Evaluation

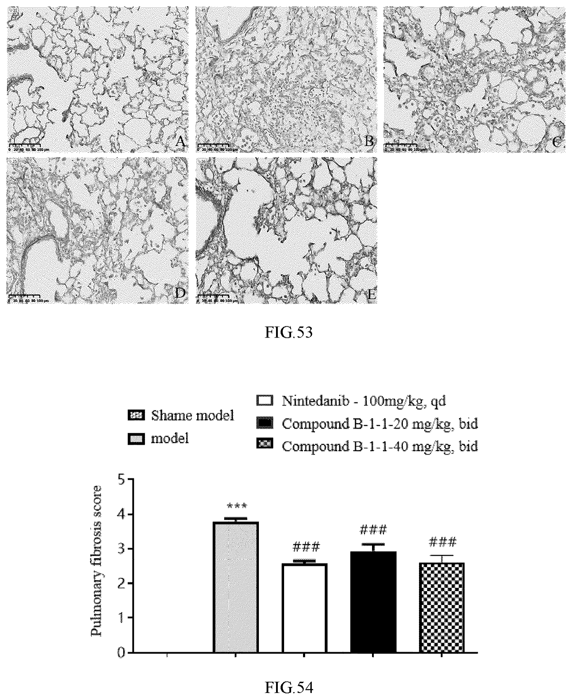

Masson staining of lung tissue revealed numerous scattered fibrotic lesions in the left lungs of animals in both the model group and all treatment groups. Pulmonary alveolar tissue exhibited varying degrees of fibrotic damage, with partial loss of alveolar architecture, thickening of the remaining alveolar walls, and deposition of fibrous tissue within alveolar spaces (Figures 52, 53).

Evaluation of the degree of fibrosis in left lung tissue was performed according to the Ashcroft scoring system (Table 4-6, Figure 54). Compared with the sham group, animals in the model group showed a significant increase in fibrosis scores (p < 0.001). Treatment with nintedanib significantly ameliorated pulmonary fibrosis compared to the model group (p < 0.001). Both doses of compound B-1-1 (20 mg/kg and 40 mg/kg) significantly reduced pulmonary fibrosis scores (p < 0.001, p < 0.001) (Table 4-6, Figure 54). In Figure 54, statistical analysis by one-way ANOVA is denoted as follows: *** p < 0.001 vs. sham group, ^{###}p< 0.001 vs. model group.

**Table 4-6 Pathological Evaluation of Pulmonary Fibrosis in the Left Lung (Mean ± SEM)**

| Group | Fibrosis Score |
|---|---|
| Sham Group | 0.00 ± 0.00 |
| Model Group | 3.79 ± 0.08 *** |
| Nintedanib 100 mg/kg, q.d. | 2.57 ± 0.09 ^{###} |
| Compound B-1-1 20 mg/kg, b.i.d. | 2.93 ± 0.20 ^{###} |
| Compound B-1-1 40 mg/kg, b.i.d. | 2.62 ± 0.18 ^{###} |

| | |
|---|---|
| Note: One-way ANOVA: *** p < 0.001 vs. Sham group; ^{###} p < 0.001 vs. Model group. Two-way ANOVA: *** p < 0.001 vs. Sham group; ^{###} p < 0.001 vs. Model group. | |

The positive control drug, nintedanib, administered once daily starting on Day 8 after model induction, significantly improved the progression of pulmonary fibrosis, demonstrated by the amelioration of pathological injuries in terminal bronchioles and accompanying small pulmonary arteries within and at the margins of fibrotic lesions, as well as by the inhibition of pulmonary fibrotic lesion progression.

Both doses (20 mg/kg and 40 mg/kg) of compound B-1-1, administered twice daily starting on Day 8 post-modeling, significantly improved pathological injury in terminal bronchioles and accompanying small pulmonary arteries within and surrounding the fibrotic lesions, and could significantly inhibit the progression of pulmonary fibrosis.

The above in vivo and in vitro pharmacodynamic studies demonstrate that compound B-1-1 not only effectively blocks the Sonic Hedgehog (Shh) signaling pathway but also downregulates the expression of fibrosis-related protein α-SMA and can significantly improve the degree of pulmonary fibrosis and idiopathic pulmonary fibrosis.

## Claims

1. Use of the compound of formula A or a pharmaceutically acceptable salt, solvate, hydrate, stereoisomer, tautomer and prodrug thereof in the preparation of a drug for treating interstitial pneumonia; 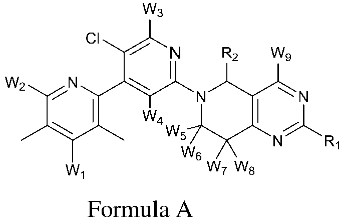 Wherein,
R₂ is C₁₋₃ alkyl, CD₃ or CF₃;
R₁ is -NRₓR_{y}, wherein Rₓ and R_{y} are each independently H, alkyl, cycloalkyl, alkylcycloalkyl, C(O)R", or -NRₓR_{y} may together form a 4-7 membered heterocyclic ring, wherein the 4-7 membered heterocyclic ring is substituted or unsubstituted; R1 is preferably substituted or unsubstituted 4-7 membered heterocyclic ring.
R" is C₁₋₅ alkyl;
W₁, W₂, W₃, W₄, W₅, W₆, W₇, W₈ and W₉ are independently H or D.

2. The use according to claim 1, wherein the compound of formula A is selected from the following compounds: 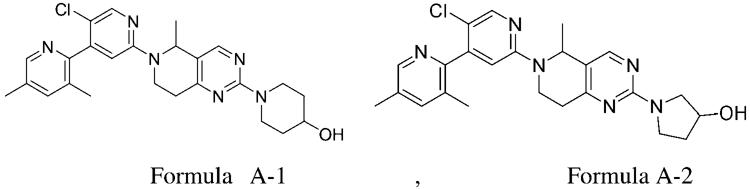 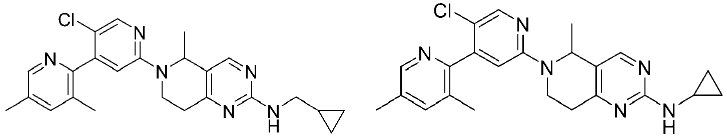 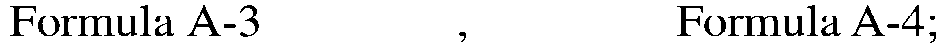 Preferably, the compound is a compound represented by formula A-1.

3. The use according to claim 1, wherein the compound of formula A is a compound having a structure of formula B: 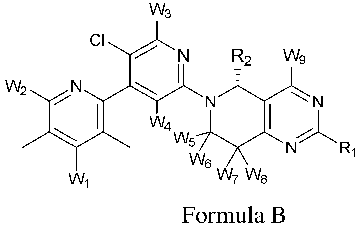
Wherein,
R₁, R₂, W₁, W₂, W₃, W₄, W₅, W₆, W₇, W₈ and W₉ are as defined in claim 1;
Preferably, the compound of formula B is a compound selected from the following compounds, preferably a compound having a structure of formula B-1: 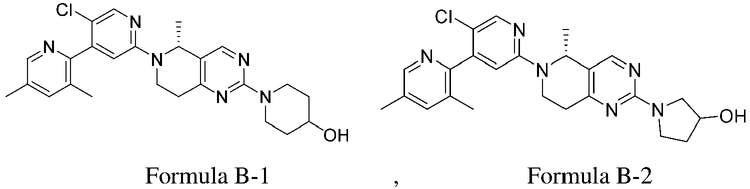 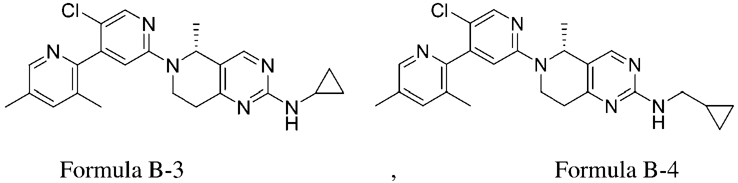 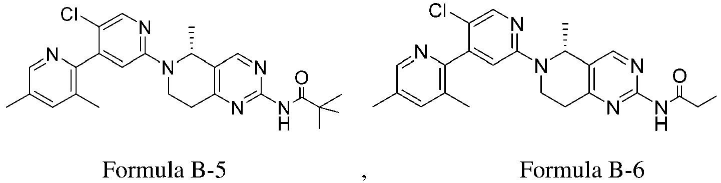
or, the compound of formula A is a compound having a structure of formula C: 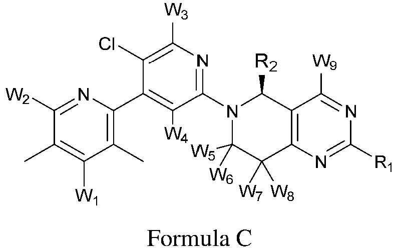
Wherein,
R₁, R₂, W₁, W₂, W₃, W₄, W₅, W₆, W₇, W₈ and W₉ are as defined in claim 1; Preferably, the compound of formula A is a compound having structure of formula C-1:

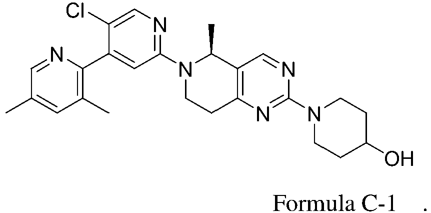

4. The use according to any one of claims 1-3, wherein the drug is a pharmaceutically acceptable salt of the compound of formula A-1, formula B-1 or formula C-1, preferably hydrochloride, succinate or fumarate of the compound of formula A-1, formula B-1 or formula C-1, more preferably fumarate or succinate of the compound of formula A-1, formula B-1, or formula C-1.

5. The use according to claim 4, wherein the drug is fumarate of the compound of formula B-1, and the molar ratio of the compound of formula B-1 to fumaric acid in fumarate of the compound of formula B-1 is 1: 0.2~1, preferably 1: 0.3 ~0.6, more preferably 1: 0.3, 1: 0.5, 1: 0.6;
or the drug is succinate salt of the compound of formula B-1, and the molar ratio of the compound of formula B-1 to succinic acid is 1: 0.3-3.0, preferably 1: 0.5-2.0, more preferably 1: 0.8-1.2.

6. The use according to claim 5, wherein the fumarate of the compound of formula B-1 has any one of the following crystal forms A-H, preferably fumarate crystal form F:
1) the fumarate of the compound of formula B-1 has a crystal form A, and the molar ratio of compound B-1 compound to fumaric acid in the crystal form A is 1: 0.5; the x-ray powder diffraction pattern thereof has characteristic peaks at 2θ values of 6.0 ± 0.2°, 12.9 ± 0.2° and 16.2 ± 0.2°; preferably, it further meets at least one of the following conditions:
i. the x-ray powder diffraction pattern has characteristic peaks at one or two or three of a 20 value of 13.3 ± 0.2°, 15.4 ± 0.2°, 19.0 ± 0.2°;
ii. the x-ray powder diffraction pattern has characteristic peaks at one or two or three of a 20 value of 12.2±0.2°, 18.1±0.2°, 20.7±0.2°;
iii. the x-ray powder diffraction pattern thereof is basically as shown in FiG.1, and/or has a diffraction peak as shown in Table 1-1;
iv. it is anhydrous;
2) the fumarate of the compound of formula B-1 has a crystal form B, and the molar ratio of the compound B-1 compound to fumaric acid in the crystal form B is 1: 0.6; the x-ray powder diffraction pattern thereof has characteristic peaks at 2θ values of 13.2 ± 0.2°, 16.0 ± 0.2° and 18.6 ± 0.2°; preferably, it further meets at least one of the following conditions:
i. the x-ray powder diffraction pattern has characteristic peaks at one or two or three of a 20 value of 12.7±0.2°, 15.4±0.2°, 16.6±0.2°;
ii. the x-ray powder diffraction pattern has characteristic peaks at one or two or three of a 20 value of 17.2±0.2°, 18.0±0.2°, 20.4±0.2°;
iii. the x-ray powder diffraction pattern thereof is basically as shown in FiG.5, and/or has a diffraction peak as shown in Table 1-2;
iv. it is anhydrous;
3) the fumarate of the compound of formula B-1 has a crystal form C, and the molar ratio of the compound B-1 compound to fumaric acid in the crystal form C is 1: 0.5; the x-ray powder diffraction pattern thereof has characteristic peaks at 2θ values of 15.1±0.2°, 17.1±0.2° and 19.4±0.2°; preferably, it further meets at least one of the following conditions:
i. the x-ray powder diffraction pattern has characteristic peaks at one or two or three of a 20 value of 11.0±0.2°, 17.8±0.2°, 21.7±0.2°;
ii. the x-ray powder diffraction pattern has characteristic peaks at one or two or three of a 20 value of 5.0±0.2°, 10.1±0.2°, 27.1±0.2°;
iii. the x-ray powder diffraction pattern thereof is basically as shown in FiG.10, and/or has a diffraction peak as shown in Table 1-3;
iv. it is anhydrous;
4) the fumarate of the compound of formula B-1 has a crystal form D, and the molar ratio of the compound B-1 compound to fumaric acid in the crystal form D is 1: 0.5; the x-ray powder diffraction pattern thereof has characteristic peaks at 2θ values of 12.7±0.2°, 17.6±0.2° and 19.1±0.2°; preferably, it further meets at least one of the following conditions:
i. the x-ray powder diffraction pattern has characteristic peaks at one or two or three of a 20 value of 6.3±0.2°, 13.2±0.2°, 16.1±0.2°;
ii. the x-ray powder diffraction pattern has characteristic peaks at one or two or three of a 20 value of 17.2±0.2°, 14.0±0.2°, 20.9±0.2°;
iii. the x-ray powder diffraction pattern thereof is basically as shown in FiG.14, and/or has a diffraction peak as shown in Table 1-4;
5) the fumarate of the compound of formula B-1 has a crystal form E, and the molar ratio of the compound B-1 compound to fumaric acid in the crystal form E is 1: 0.5; the x-ray powder diffraction pattern thereof has characteristic peaks at 2θ values of 15.2±0.2°,16.0±0.2° and 22.0±0.2°; preferably, it further meets at least one of the following conditions:
i. the x-ray powder diffraction pattern has characteristic peaks at one or two or three of a 20 value of 12.8±0.2°, 20.5±0.2°, 26.0±0.2°;
ii. the x-ray powder diffraction pattern has characteristic peaks at one or two or three of a 20 value of 16.8±0.2°, 18.1±0.2°, 19.7±0.2°;
iii. the x-ray powder diffraction pattern thereof is basically as shown in FiG.21, and/or has a diffraction peak as shown in Table 1-5;
6) the fumarate of the compound of formula B-1 has a crystal form F, and the molar ratio of the compound B-1 compound to fumaric acid in the crystal form F is 1: 0.5; the x-ray powder diffraction pattern thereof has characteristic peaks at 2θ values of 11.1±0.2°, 16.6±0.2° and 22.4±0.2°; preferably, it further meets at least one of the following conditions:
i. the x-ray powder diffraction pattern has characteristic peaks at one or two or three of a 2θ value of 16.9±0.2°, 18.6±0.2°, 21.0±0.2°;
ii. the x-ray powder diffraction pattern has characteristic peaks at one or two or three of a 2θ value of 5.5±0.2°, 14.5±0.2°, 25.0±0.2°;
iii. the x-ray powder diffraction pattern thereof is basically as shown in FiG.26, and/or has a diffraction peak as shown in Table 1-6;
iv. it is anhydrous;
7) the fumarate of the compound of formula B-1 has a crystal form G, the x-ray powder diffraction pattern thereof has characteristic peaks at 2θ values of 15.6±0.2°, 17.7±0.2°, 20.6±0.2°; preferably, it further meets at least one of the following conditions:
i. the x-ray powder diffraction pattern thereof is basically as shown in FiG.31, and/or has a diffraction peak as shown in Table 1-10;
ii. the molar ratio of the compound B-1 compound to fumaric acid in the crystal form G is 1: 0.3;
8) the fumarate of the compound of formula B-1 has a crystal form H, and the molar ratio of the compound B-1 compound to fumaric acid in the crystal form H is 1: 0.5; the x-ray powder diffraction pattern thereof has characteristic peaks at 2θ values of 12.4±0.2°, 16.2±0.2° and 21.8±0.2°; preferably, it further meets at least one of the following conditions:
i. the x-ray powder diffraction pattern has characteristic peaks at one or two or three of a 20 value of 12.8±0.2°, 15.8±0.2°, 18.0±0.2°;
ii. the x-ray powder diffraction pattern has characteristic peaks at one or two or three of a 20 value of 14.5±0.2°, 16.9±0.2°, 21.2±0.2°;
iii. the x-ray powder diffraction pattern thereof is basically as shown in FiG.35, and/or has a diffraction peak as shown in Table 1-11.

7. The use according to claim 5, wherein the succinate of the compound of formula B-1 has any one of the following crystal forms:
1) the succinate of the compound of formula B-1 has a crystal form A, and the x-ray powder diffraction pattern thereof has characteristic peaks at 2θ values of 22.1 ± 0.2° and 27.2 ± 0.2°;
preferably, the x-ray powder diffraction pattern thereof has a diffraction peak substantially as shown in Table 2-1;
2) the succinate of the compound of formula B-1 has a crystal form B, the x-ray powder diffraction pattern thereof has characteristic peaks at 2θ values of 9.5±0.2°, 14.4±0.2° and 23.6±0.2°; preferably, it further meets at least one of the following conditions:
i. the x-ray powder diffraction pattern has characteristic peaks at one or two or three of a 2θ value of 15.1±0.2°, 19.2±0.2°, 25.5±0.2°;
ii. the x-ray powder diffraction pattern has characteristic peaks at one or two or three of a 2θ value of 15.6±0.2°, 22.3±0.2°, 24.0±0.2°;
iii. the x-ray powder diffraction pattern thereof has a diffraction peak substantially as shown in Table 2-2.

8. The use according to any one of claims 1-7, wherein the interstitial pneumonia is idiopathic interstitial pneumonias, preferably idiopathic pulmonary fibrosis, desquamative interstitial pneumonia, nonspecific interstitial pneumonia, cryptogenic organometallic pneumonia, respiratory bronchiolitis-associated interstitial lung disease, lymphocyte interstitial pneumonia, more preferably idiopathic pulmonary fibrosis.

9. A pharmaceutical composition comprising an effective amount of the compound or pharmaceutically acceptable salt, solvate, hydrate, stereoisomer, tautomer and prodrug thereof for use according to claims 1 to 7, and a pharmaceutically acceptable carrier.

10. A combined application composition comprising: (i) any one or more of N-acetylcysteine, pirfenidone, nintedanib, glucocorticoids (such as methylprednisolone, prednisone, fluticasone), immunosuppressants (such as cyclosporine, cyclophosphamide, Azathioprine), endothelin receptor antagonists (such as Macitentan, Bosentan, Ambrisentan ), JNK Inhibitors ( Tanzisertib (CC -930)), Itraconazole, Vismodegib, hydroxycarbamide, Danazol, Amikacin, dornase alfa, Tobramycin, Tiotropium bromide, Treprostinil, Zileuton, Saridegib ( IPI -926 ), cisplatin, paclitaxel, camptothecin, trastuzumab, Gleevec, imatinib, gefitinib, erlotinib, and lapatinib; and (ii) compound of formula A or the pharmaceutically acceptable salt, solvate, hydrate, stereoisomer, tautomer, or prodrug thereof.

11. A drug for treating interstitial pneumonia, comprising the compound or pharmaceutically acceptable salt, solvate, hydrate, stereoisomer, tautomer, and prodrug thereof for use according to any one of claims 1 to 7, or the pharmaceutical composition according to any one of claims 9 to 10.

12. A method of treating interstitial pneumonia comprising administering to a subject in need thereof a therapeutically effective amount of the compound or pharmaceutically acceptable salt, solvate, hydrate, stereoisomer, tautomer, and prodrug thereof for use according to any one of claims 1 to 7, or the pharmaceutical composition according to any one of claims 9 to 10, preferably interstitial pneumonia is idiopathic interstitial pneumonias, more preferably idiopathic pulmonary fibrosis.
